# FASCICULE DE BREVET EUROPEEN

(11) **EP 1 259 606 B1**
(45) Date de publication et mention de la délivrance du brevet: **28.04.2010**
(21) Numéro de dépôt: 01907820.3
(22) Date de dépôt: 15.02.2001
(51) Int. Cl.: C12N 15/12, C12N 5/10, C07K 14/47, C07K 16/18, G01N 33/50, A61K 38/17

(54) **COMPOSITIONS UTILISABLES POUR REGULER L'ACTIVITE DE LA PARKINE**
ZUR STEUERUNG DER PARKINAKTIVITÄT BRAUCHBARE ZUSAMMENSETZUNGEN
COMPOSITIONS USEFUL FOR REGULATING PARKIN GENE ACTIVITY

(30) Priorité: 17.02.2000 FR 0001980; 18.04.2000 US 198489 P
(43) Date de publication de la demande: 27.11.2002
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR)
(72) Inventeur: KOUTNIKOVA, Hana, F-67540 Ostwald (FR); BRICE, Alexis, F-75006 Paris (FR); FOURNIER, Alain, F-92290 Châtenay Malabry (FR); PRADIER, Laurent, F-91370 Verrières (FR); PRADES, Catherine, F-94320 Thiais (FR); ARNOULD-REGUIGNE, Isabelle, F-94430 Chennevières sur Marne (FR); ROSIER-MONTUS, Marie-Françoise, F-92160 Antony (FR); CORTI, Olga, F-75013 Paris (FR)
(74) Mandataire: Bouvet, Philippe
(86) Numéro de dépôt international: PCT/FR2001/000461
(87) Numéro de publication internationale: WO 2001/060857

(56) Documents cités:
- WO-A-01/46256
- WO-A-01/53312
- DATABASE EMBL SEQUENCES [en ligne] Accession No. AI701008, 4 juin 1999 (1999-06-04) STRAUSBERG R.: "EST; H. sapiens cDNA clone IMAGE:2340603 similar to TR:Q14184 DOC2-beta." XP002152292
- DATABASE EMBL SEQUENCES [en ligne] ACCESSION NO. AB050743, FUKUDA M.: "Mus musculus slp-3b" XP002175418 -& FUKUDA M. & MIKOSHIBA K.: "Synaptotagmin-like protein 1-3: A novel family of C-terminal-type tandem C2 proteins." BIOCHEM. BIOPHYS. RES. COM., vol. 281, mars 2001 (2001-03), pages 1226-1233, XP002175417
- KITADA ET AL: "Mutations in the parkin gene cause autosomal recessive juvenile parkinsonism" NATURE,GB,MACMILLAN JOURNALS LTD. LONDON, vol. 392, no. 6676, 9 avril 1998 (1998-04-09), pages 605-608, XP002108469 ISSN: 0028-0836 cité dans la demande

## Description

La présente invention concerne des compositions et méthodes utilisables pour réguler l'activité de la parkine. Elle concerne notamment une nouvelle protéine, désignée PAP1, partenaire de la parkine, ainsi que les peptides ou polypeptides dérivés ou homologues de cette protéine. Elle concerne également des composés capables de moduler au moins partiellement l'activité de la Parkine, notamment d'interférer avec l'interaction entre la parkine et PAP1. La présente invention est utilisable dans les domaines thérapeutiques, diagnostiques ou pour 1a constitution de cibles pharmacologiques permettant le développement de nouveaux médicaments.

Le gène de la Parkine est muté dans certaines formes familiales (juvéniles autosomiques récessives) de la maladie de Parkinson (Kitada *et al*., 1998). La maladie de Parkinson (Lewy, 1912) est une des maladies neurodégénératives les plus communes, affectant plus de 1% de la population de plus de 55 ans. Les patients atteints de cette maladie ont des troubles neurologiques rassemblés sous le terme de syndrome parkinsonien, caractérisé par une rigidité, une bradykinésie, et un tremblement de repos. Ces symptômes sont la conséquence d'une dégénérescence des neurones dopaminergiques de la substance noire du cerveau.

La plupart des cas avec une maladie de Parkinson n'ont pas d'histoire familiale. Cependant, il existe des cas familiaux dont certains correspondent à une forme monogénique de la maladie. A l'heure actuelle, seulement trois gènes différents ont été identifiés dans certaines formes héréditaires rares. La première forme correspond à une forme autosomique dominante dont le gène responsable code pour l'alpha Synucléine (Polymeropoulos *et al*., 1997). Cette protéine est un constituant abondant des inclusions intracytoplamiques, appelées corps de Lewy, qui servent de marqueur de la maladie de Parkinson (Lewy, 1912). La seconde forme, également autosomique dominante, est liée à une mutation dans un gène codant pour une hydrolase appelée ubiquitin carboxy-terminal hydrolase L1 (Leroy *et al*., 1998). Cette enzyme est supposée hydrolyser les polymères ou les conjugués d'ubiquitines en monomères d'ubiquitine. La troisième forme se distingue des précédentes par une transmission autosomique récessive et un début souvent avant 40 ans ainsi que par une absence de corps de Lewy. Ces malades répondent plus favorablement à la levodopa, un précurseur de la dopamine utilisé comme traitement de la maladie de Parkinson. Le gène impliqué dans cette forme code pour une nouvelle protéine appelée Parkine (Kitada *et al*., 1998).

Le gène de la Parkine est constitué de 12 exons couvrant une région génomique de plus de 500 000 paires de bases sur le chromosome 6 (6q25.2-q27). A l'heure actuelle, deux grands types de mutation de ce gène, à l'origine de la maladie, sont connus, soit des délétions de taille variable dans la région qui couvre les exons 2 à 9, soit des mutations ponctuelles qui produisent l'apparition prématurée d'un codon stop ou le changement d'un acide aminé (Kitada *et al*., 1998; Abbas *et al*., 1999; Lucking *et al*., 1998; Hattori *et al*., 1998). La nature de ces mutations et le mode de transmission autosomique récessif suggèrent une perte de fonction de la Parkine conduisant à la maladie de Parkinson.

Ce gène est exprimé dans un grand nombre de tissus et notamment dans la substance noire. Il existe plusieurs transcrits correspondant à ce gène qui proviennent d'épissages alternatifs différents (Kitada *et al*., 1998; Sunada *et al*., 1998). Dans le cerveau, on trouve deux types d'ARN messagers dont un est dépourvu de la partie correspondant à l'exon 5. Dans les leucocytes, des ARN messagers de la Parkine ne contenant pas la région codant pour les exons 3, 4 et 5 ont été identifiés. Le plus long des ARN messagers de la Parkine, présent dans le cerveau, contient 2960 bases et code pour une protéine de 465 acides aminés.

Cette protéine a une faible homologie dans sa partie N-terminale avec l'ubiquitine. Sa moitié C-terminale contient deux motifs « ring finger » séparés par un domaine IBR (In Between Ring) correspondant à une région riche en cystéine pouvant lier des métaux comme les domaines «zing finger» (Morett, 1999). Par immunocytochimie, il a été montré que la Parkine est localisée dans le cytoplasme et l'appareil de Golgi de neurones de la substance noire qui contiennent de la mélanine (Shimura *et al*., 1999). De plus, cette protéine est présente dans certains corps de Lewy de Parkinsoniens. La fonction cellulaire de la Parkine n'est pas encore démontrée, mais elle pourrait jouer un rôle de transporteur dans les vésicules synaptiques, dans la maturation ou dégradation des protéines et dans le contrôle de la croissance, de la différenciation ou du développement cellulaire. Dans les formes autosomiques récessives juvéniles, la Parkine est absente, confirmant ainsi que la perte de cette fonction est responsable de la maladie.

L'élucidation du rôle exact de la protéine Parkine dans le processus de dégénérescence des neurones dopaminergiques constitue donc un enjeu majeur pour la compréhension et l'approche thérapeutique de la maladie de Parkinson et plus généralement des maladies du système nerveux central.

La présente invention réside dans l'identification d'un partenaire de la parkine, interagissant avec cette protéine dans des conditions physiologiques. Ce partenaire représente une nouvelle cible pharmacologique pour la fabrication ou la recherche de composés capables de moduler l'activité de la parkine, notamment son activité sur la dégénérescence des neurones dopaminergiques et/ou le développement de pathologies nerveuses. Cette protéine, les anticorps, les acides nucléiques correspondant ainsi que les sondes ou amorces spécifiques, sont également utilisables pour la détection ou le dosage des protéines dans des échantillons biologiques, notamment des échantillons de tissu nerveux. Ces protéines ou acides nucléiques sont également utilisables dans des approches thérapeutiques, pour moduler l'activité de la parkine, ainsi que tout composé selon l'invention, capables de moduler l'interaction entre la parkine et les polypeptides de l'invention.

La présente invention résulte plus particulièrement de la mise en évidence par la demanderesse d'une nouvelle protéine humaine, désignée PAP1 (Parkine Associated Protein 1) ou LY111, interagissant avec la parkine. La protéine PAP1 (séquence SEQ ID NO:1 ou 2) présente une certaine homologie avec les synaptotagmines et est capable d'interagir plus particulièrement avec la région centrale de la parkine (représentée sur la séquence SEQ ID NO :3 ou 4). La protéine PAP1 a également été clonée, séquencée et caractérisée à partir de différents tissus d'origine humaine, notamment de poumon (SEQ ID NO : 12, 13) et de cerveau (SEQ ID NO : 42, 43), ainsi que des formes courtes, correspondant à des variants d'épissage (SEQ ID NO : 14, 15, 44, 45).

La présente invention résulte également de l'identification et de la caractérisation de régions particulières de la protéine PAP1, impliquées dans la modulation de la fonction de la parkine. La mise en évidence de l'existence de cette protéine et de régions impliquées dans sa fonction permet notamment de préparer de nouveaux composés et/ou compositions utilisables comme agents pharmaceutiques et de développer des méthodes industrielles de criblage de tels composés.

Un premier objet de l'invention concerne un polypeptide interagissant avec la proteine présentant surtoute sa longeur une identité d'au moins 95 % avec la s~equence SEQ IDN°2.

Un autre aspect de l'invention réside dans un acide nucléique codant la protéine PAP1, ses fragments, dérivés ou homologues, ainsi que tout vecteur comprenant un tel acide nucléique, toute cellule recombinante contenant un tel acide nucléique ou vecteur et tout mammifère non-humain comprenant dans ses cellules un tel acide nucléique.

L'invention concerne aussi des anticorps capables de lier la protéine PAP1, ses fragments, dérivés et homologues, notamment des anticorps polyclonaux ou monoclonaux, plus préférentiellement des anticorps capables de lier la protéine PAP1 et d'inhiber au moins partiellement son interaction avec la parkine.

Un autre aspect de l'invention concerne des sondes ou amorces nucléotidiques, spécifiques de PAP1, utilisables pour détecter ou amplifier le gène de papl ou une région de celui-ci dans tout échantillon biologique.

L'invention concerne encore des compositions pharmaceutiques, des méthodes de détection d'anomalies génétiques, des méthodes de production de polypeptides tels que définis ci-avant, ainsi que des méthodes de criblage ou de caractérisation de composés actifs.

Au sens de la présente invention, la dénomination protéine PAP1 désigne la protéine en soit ainsi que toutes ses formes homologues. Par forme homologue on entend désigner toutes protéines équivalentes à la protéine considérée, d'origine cellulaire diverse et notamment issue de cellules d'origine humaine, ou d'autres organismes, et possédant une activité de même type. De tels homologues comprennent également les variants naturels de la protéine PAP1 de séquence SEQ ID NO2, notamment les variants polymorphiques ou d'épissage. De tels homologues peuvent être obtenus par des expériences d'hybridation entre les acides nucléiques codant (notamment l'acide nucléique de séquence SEQ ID NO:1). Au sens de l'invention, il suffit qu'une séquence de ce type présente un pourcentage d'identité significatif pour conduire à un comportement physiologique assimilable à celui de la protéine PAP1 tel que revendiquée. A cet égard, des variants et/ou homologues de la séquence SEQ ID NO : 2 sont décrits dans les séquences SEQ ID NO : 13, 15, 43 et 45, identifiés à partir de tissus d'origine humaine. La dénomination PAP1 englobe donc également ces polypeptides. Le " pourcentage d'identité " entre deux séquences de nucléotides ou d'acides aminés, au sens de la présente invention, peut être déterminé en comparant deux séquences alignées de manière optimale, à travers une fenêtre de comparaison.

La partie de la séquence nucléotidique ou polypeptide dans la fenêtre de comparaison peut ainsi comprendre des additions ou des délétions (par exemple des " gaps ") par rapport à la séquence de référence (qui ne comprend pas ces additions ou ces délétions) de manière à obtenir un alignement optimal des deux séquences.

Le pourcentage est calculé en déterminant le nombre de positions auquel une base nucléique ou un résidu d'aminoacide identique est observé pour les deux séquences (nucléique ou peptidique) comparées, puis en divisant le nombre de positions auquel il y a identité entre les deux bases ou résidus d'aminoacides par le nombre total de positions dans la fenêtre de comparaison, puis en multipliant le résultat par 100 afin d'obtenir le pourcentage d'identité de séquence.

L'alignement optimal des séquences pour la comparaison peut être réalisé de manière informatique à l'aide d'algorithmes connus contenus dans le package de la Société WISCONSIN GENETICS SOFTWARE PACKAGE, GENETICS COMPUTER GROUP (GCG), 575 Science Doctor, Madison, WISCONSIN.

À titre d'illustration, le pourcentage d'identité de séquence pourra être effectué à l'aide du logiciel BLAST (versions BLAST 1.4.9 de mars 1996. BLAST 2.0.4 de février 1998 et BLAST 2.0.6 de septembre 1998), en utilisant exclusivement les paramètres par défaut (Altschul et al, J. Mol. Biol., (1990) 215 : 403-410 ; Altschul et al, Nucleic Acids Res. (1997) 25 : 3389-3402). Blast recherche des séquences similaires/homologues à une séquence " requête " de référence, à l'aide de l'algorithme d'Altschul et al. (Supra). La séquence requête et les bases de données utilisées peuvent être peptidiques ou nucléiques, toute combinaison étant possible.

Au sens de la présente invention, le terme dérivé désigne toute séquence différant de la séquence considérée en raison d'une dégénérescence du code génétique, obtenue par une ou plusieurs modifications de nature génétique et/ou chimique, ainsi que tout peptide codé par une séquence hybridant avec la séquence nucléique SEQ ID NO : 1 ou un fragment de celle-ci, par exemple avec la séquence nucléique SEQ ID NO : 12, 14, 42 ou 44 ou un fragment de celles-ci, et présentant la capacité d'interférer au niveau de l'interaction entre là protéine PAP1, ou l'un de ses homologues, et la Parkine. Par modification de nature génétique et/ou chimique, on peut entendre toute mutation, substitution, délétion, addition et/ou modification d'un ou plusieurs résidus. Le terme dérivé comprend également les séquences homologues à la séquence considérée, issues d'autres sources cellulaires et notamment de cellules d'origine humaine, ou d'autres organismes, et possédant une activité de même type. De telles séquences homologues peuvent être obtenues par des expériences d'hybridation. Les hybridations peuvent être réalisées à partir de banques d'acides nucléiques, en utilisant comme sonde la séquence native ou un fragment de celle-ci, dans des conditions variables d'hybridation (Maniatis *et al*., 1989). Par ailleurs, le terme « fragment » ou « partie » désigne toute portion de la molécule considérée, comprenant au moins 5 résidus consécutifs, de préférence au moins 9 résidus consécutifs, encore plus préférentiellement au moins 15 résidus consécutifs. Des fragments typiques peuvent comprendre au moins 25 résidus consécutifs.

De tels dérivés ou fragments peuvent être générés dans des buts différents, tels que notamment celui d'augmenter leur efficacité thérapeutique ou de réduire leurs effets secondaires, ou celui de leur conférer de nouvelles propriétés pharmacocinétiques et/ou biologiques.

Un objet spécifique de la présente invention concerne la protéine PAP1 de séquence SEQ ID NO : 13 ou 43.

Un autre objet de l'invention réside dans des anticorps ou fragments ou dérivés d'anticorps polyclonaux ou monoclonaux dirigés contre un polypeptide tel que défini ci-avant. De tels anticorps peuvent être générés par des méthodes connues de l'homme du métier. En particulier, ces anticorps peuvent être préparés par immunisation d'un animal contre un composé peptidique de l'invention (notamment un polypeptide ou un peptide comprenant tout ou partie de la séquence SEQ ID NO :2), prélèvement du sang, et isolement des anticorps. Ces anticorps peuvent également être générés par préparation d'hybridomes selon les techniques connues de l'homme de l'art.

Plus préférentiellement, les anticorps ou fragments d'anticorps de l'invention présentent la capacité de moduler au moins partiellement l'interaction des peptides revendiqués avec la Parkine.

Par ailleurs, ces anticorps peuvent également être utilisés pour détecter et/ou doser l'expression de la PAP1 dans des échantillons biologiques, et de ce fait, pour renseigner sur son état d'activation.

Les fragments ou dérivés d'anticorps sont par exemple des fragments Fab, Fab'2, des anticorps simple-chaine (ScFv), etc. Il s'agit notamment de tout fragment ou dérivé conservant la spécificité antigénique des anticorps dont ils sont issus.

Les anticorps selon l'invention sont plus préférentiellement capables de lier les protéines PAP1 comprenant la séquence SEQ ID NO :2, 13, 15, 43 ou 45, notamment la région de cette protéine impliquée dans l'interaction avec la parkine. Ces anticorps (ou fragments ou dérivés) sont plus préférentiellement capables de lier un épitope présent dans la séquence comprise entre les résidus 1 et 344 de la séquence SEQ ID NO :2.

L'invention concerne également les composés non peptidiques ou non exclusivement peptidiques utilisables comme agent pharmaceutique. Il est en effet possible, à partir des motifs protéiques actifs décrits dans la présente demande, de réaliser des molécules modulatrices de l'activité de PAP1 non exclusivement peptidiques et compatibles avec une utilisation pharmaceutique, en particulier en dupliquant les motifs actifs des peptides avec une structure non peptidique ou de nature non exclusivement peptidique.

La présente invention a également pour objet tout acide nucléique codant pour un composé peptidique selon l'invention. Il peut s'agir en particulier d'un acide nucléique comprenant tout ou partie de la séquence SEQ ID N°1,12, 14, 42 ou 44, ou un de ses derivés. Par séquence dérivée, on entend au sens de la présente invention toute séquence hybridant avec la séquence présentée en SEQ ID N°1 ou avec un fragment de celle-ci et codant pour un composé peptidique selon l'invention, ainsi que les séquences résultant de ces dernières par dégénérescence du code génétique. Des acides nucléiques selon l'invention comportent par exemple tout ou partie de la séquence nucléique SEQ ID NO : 12, 14, 42 ou 44.

La présente invention est en outre relative à des séquences présentant un pourcentage d'identité significatif avec la séquence présentée en SEQ ID N°1 ou avec un fragment de celle-ci et codant pour un composé peptidique présentant un comportement physiologique assimilable à celui de la protéine PAP1. On entend par pourcentage d'identité significatif un pourcentage d'au moins 60%, préférentiellement de 80%, plus préférentiellement de 90% et encore plus préférentiellement de 95%.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues soit par criblage de banques d'ADN (banque d'ADNc, banque d'ADN génomique), soit par synthèse chimique, soit par des méthodes mixtes incluant la modification chimique ou enzymatique de séquences obtenues par criblage de banques, soit par recherche d'homologie dans des bases de données nucléiques ou protéiques. L'hybridation mentionnée ci-dessus est préférentiellement réalisée dans les conditions décrites par Sambrook et al (1989, pages 9.52-9.55).
Elle est avantageusement réalisée dans des conditions d'hybridation de forte stringence. Par " conditions d'hybridation de forte stringence " au sens de la présente invention, on entendra les conditions suivantes :

### 1- Compétition des membranes et PRE HYBRIDATION :

- Mélanger : 40µl ADN sperme de saumon (10mg/ml)
   + 40 µl ADN placentaire humain (10mg/ml)
- Dénaturer 5 mn à 96°C, puis plonger dans la glace le mélange.
- Oter le tampon SSC 2X et verser 4 ml de mix formamide dans le tube d'hybridation contenant les membranes.
- Ajouter le mélange des deux ADNs dénaturés.
- Incubation à 42°C pendant 5 à 6 heures, avec rotation.

### 2- Compétition de la sonde maquée :

- Ajouter à la sonde marquée et purifiée 10 à 50 µl ADN Cot I, selon la quantité d'hybridations non spécifiques.
- Dénaturer 7 à 10 mn à 95°C.
- Incuber à 65°C pendant 2 à 5 heures.

### 3- Hybridation:

- Oter le mix de pré hybridation.
- Mélanger 40 µl ADN sperme de saumon + 40 µl ADN placentaire humain ; dénaturer 5 mn à 96°C, puis plonger dans la glace.
- Ajouter dans le tube d'hybridation 4 ml de mix formamide, le mélange des deux ADN et la sonde marquée/ADN Cot I dénaturée.
- Incuber 15 à 20 heures à 42°C, avec rotation.

### 4- Lavages :

- Un lavage à température ambiante dans du SSC 2X, pour rincer.
- 2 fois 5 minutes à température ambiante SSC 2X et SDS 0,1%.
   - 2 fois 15 minutes SSC 0,1X et SDS 0,1% à 65°C.
Envelopper les membranes dans du Saran et exposer.

Les conditions d'hybridation décrites plus haut sont adaptées à l'hybridation dans des conditions de forte stringence, d'une molécule d'acide nucléique d'une longueur variable de 20 nucléotides à plusieurs centaines de nucléotides.

Il va sans dire que les conditions d'hybridation ci-dessus décrites peuvent être adaptées en fonction de la longueur de l'acide nucléique dont l'hybridation est recherchée ou du type de marquage choisi, selon des techniques connues de l'homme du métier.

Les conditions convenables d'hybridation peuvent par exemple être adaptées selon l'enseignement contenu dans l'ouvrage de HAMES et HIGGINS (1985) (Nucleic acid Hybridization iapractical Approach, Hames and Higgins Ed., IRL Press, Oxford) ou encore dans l'ouvrage de F. AUSUBEL et al (1999) (Currents Protocols in Molecular Biology, Green Publishing Associates and Wiley Interscience, N.Y).

Un acide nucléique particulier au sens de l'invention code pour un polypeptide comprenant la séquence SEQ ID NO :2 ou un fragment ou dérivé de celle-ci, notamment pour la protéine PAP1 humaine. Il s'agit avantageusement d'un acide nucléique comprenant la séquence nucléique SEQ ID NO :1, 12, 14, 42 ou 44.

De tels acides nucléiques peuvent être utilisés pour la production des composés peptidiques de l'invention. La présente demande concerne ainsi un procédé de préparation de tels composés peptidiques selon lequel on cultive une cellule contenant un acide nucléique selon l'invention, dans des conditions d'expression dudit acide nucléique et on récupère le composé peptidique produit. Dans ce cas, la partie codant pour ledit composé peptidique est généralement placée sous le contrôle de signaux permettant son expression dans un hôte cellulaire. Le choix de ces signaux (promoteurs, terminateurs, séquence "leader" de sécrétion, etc) peut varier en fonction de l'hôte cellulaire utilisé. Par ailleurs, les acides nucléiques de l'invention peuvent faire partie d'un vecteur qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur. Il peut s'agir d'un vecteur de type plasmidique, épisomique, chromosomique, viral, etc.

Les hôtes cellulaires utilisables pour la production des composés peptidiques de l'invention par voie recombinante sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces*, *Kluyveromyces*, *Pichia*, *Schwanniomyces,* ou *Hansenula.* S'agissant de cellules animales, on peut citer les cellules COS, CHO, C127, PC12 etc. Parmi les champignons, on peut citer plus particulièrement *Aspergillus* ssp. ou *Trichoderma* ssp. Comme hôtes procaryotes, on préfère utiliser les bactéries suivantes *E. coli, Bacillus,* ou *Streptomyces.*

La présente invention a en outre pour objet des mammifères non-humains comprenant dans leurs cellules un acide nucléique ou un vecteur selon l'invention.

De tels mammifères (rongeurs, canins, lapins, etc.) sont notamment utilisables pour l'étude des propriétés de la PAP1 et l'identification de composés à visée thérapeutique. La modification du génome d'un tel animal transgénique peut résulter d'une altération ou une modification de un ou plusieurs gènes par "knock-in" ou par "knock-out". Cette modification peuventt être effectuées à l'aide d'agents altérants ou mutagènes classiques ou bien par mutagénèse dirigée. La modification du génome peut également résulter d'une insertion de gène(s) ou de remplacement de gène(s) dans sa (leur) forme sauvage ou mutée. Les modifications du génome sont avantageusement effectuées sur des cellules souches reproductrices et avantageusement sur les pronucléi. La transgénèse peut être effectuée par microinjection d'une cassette d'expression comprenant les gènes modifiés dans les deux pronucléi fécondés. Ainsi un animal selon la présente invention peut être obtenu par injection d'une cassette d'expression comprenant un acide nucléique. De manière préférentielle, cet acide nucléique est un ADN qui peut être un ADN génomique (ADNg) ou un ADN complémentaire (ADNc).
La construction d'animaux transgéniques selon l'invention peut être réalisée selon des techniques classiques bien connues de l'homme du métier. L'homme du métier pourra en particulier se référer à la production d'animaux transgéniques, et particulièrement à la production de souris transgéniques, telles que décrites dans les brevets US 4,873,191, US 5,464,764 et US 5,789,215, le contenu de ces documents étant ici incorporé par référence.

En bref, une construction polynucléotidique comprenant un acide nucléique selon l'invention est insérée dans une lignée de cellules souches de type ES. L'insertion de la construction polynucléotidique est réalisée de préférence par électroporation, telle que décrite par Thomas et al. (1987, Cell, Vol.51:503-512).

Les cellules ayant subi l'étape d'électroporation sont ensuite criblées pour la présence de la construction polynucléotidique (par exemple par sélection à l'aide de marqueurs, ou encore par PCR ou par analyse sur gel d'électrophorèse d'ADN de type Southem) afin de sélectionner les cellules positives ayant intégré la construction polynucléotidique exogène dans leur génome, le cas échéant à la suite d'un événement de recombinaison homologue. Une telle technique est par exemple décrite par MANSOUR et al. (Nature (1988) 336: 348-352).

Ensuite, les cellules sélectionnées positivement sont isolées, clonées et injectées dans des blastocystes de souris de 3,5 jours, comme cela est décrit par BRADLEY (1987, Production and Analysis of Chimaeric mice. In: E. J. ROBERTSON (Ed., teratocarcinomas and embryonic stem cells: A practical approach. IRL press, Oxford, page 113). Des blastocystes sont ensuite introduits dans un animal hôte femelle et le développement de l'embryon est poursuivi jusqu'au terme.

Selon une alternative, des cellules de type ES sélectionnées positivement sont mises en contact avec des embryons de 2,5 jours à un stade 8-16 cellules (*morulae*) comme décrit par WOOD et al. (1993, Proc. Natl. Acad. Sci. USA, vol.90: 4582-4585) ou par NAGY et al. (1993, Proc. Natl. Acad. Sci. USA, vol. 90: 8424-8428), les cellules ES étant intemalisées afin de coloniser extensivement le blastocyste, y compris les cellules donnant naissance à la lignée germinale.

Les descendants sont ensuite testés afin de déterminer ceux qui ont intégré la construction polynucléotidique (le transgène).

Les acides nucléiques selon l'invention peuvent également servir à la réalisation d'oligonucléotides antisens ou d'antisens génétiques utilisables comme agents pharmaceutiques. Les séquences antisens sont des oligonucléotides de petite taille, complémentaire du brin codant d'un gène donné, et de ce fait capables d'hybrider spécifiquement avec l'ARNm transcrit, inhibant sa traduction en protéine. L'invention a ainsi pour objet les séquences antisens capables d'inhiber au moins partiellement l'interaction des protéines PAP1 sur la Parkine. De telles séquences peuvent être constituées par tout ou partie des séquences nucléiques définies ci-avant. Il s'agit généralement de séquences ou de fragments de séquences complémentaires de séquences codant pour des peptides interagissant avec la Parkine. De tels oligonucléotides peuvent être obtenus par fragmentation, etc, ou par synthèse chimique.

Les séquences revendiquées peuvent être utilisées dans le cadre de thérapies géniques, pour le transfert et l'expression *in vivo* de séquences antisens ou de peptides capables de moduler l'interaction de la protéine PAP1 avec la Parkine. A cet égard, les séquences peuvent être incorporées dans des vecteurs viraux ou non viraux, permettant leur administration *in vivo* (Kahn *et al*., 1991). A titre de vecteurs viraux conformes à l'invention on peut tout particulièrement citer les vecteurs de type adénovirus, rétrovirus, virus associé à l'adénovirus (AAV) ou virus de l'herpès. La présente demande a également pour objet des virus recombinants défectifs comprenant un acide nucléique codant pour un polypeptide selon l'invention, notamment un polypeptide ou peptide comprenant tout ou partie de la séquence SEQ ID NO :2 ou d'un dérivé de celle-ci, par exemple tout ou partie de la séquence SEQ ID NO : 12, 14, 42 ou 44 ou de dérivées de celles-ci.

L'invention permet également la réalisation de sondes nucléotidiques, synthétiques ou non, capables de s'hybrider avec les séquences nucléotidiques définies ci-avant ou leur brin complémentaire. De telles sondes peuvent être utilisées *in vitro* comme outil de diagnostic, pour la détection de l'expression ou surexpression de PAP1, ou encore pour la mise en évidence d'anomalies génétiques (mauvais épissage, polymorphisme, mutations ponctuelles, etc). Ces sondes peuvent également être utilisées pour la mise en évidence et l'isolement de séquences d'acides nucléiques homologues codant pour des peptides tels que définis précédemment, à partir d'autres sources cellulaires et préférentiellement de cellules d'origines humaines. Les sondes de l'invention comportent généralement au moins 10 bases, et elles peuvent par exemple comporter jusqu'à l'intégralité d'une des séquences précitées ou de leur brin complémentaire. Préférentiellement, ces sondes sont marquées préalablement à leur utilisation. Pour cela, différentes techniques connues de l'homme du métier peuvent être employées (marquage radioactif, fluorescent, enzymatique, chimique, etc).

L'invention concerne également des amorces ou couples d'amorces permettant d'amplifier tout ou une partie d'un acide nucléique codant une PAP1, par exemple une amorce de séquence choisie parmi SEQ ID NO : 16-41.

L'invention a encore pour objet toute composition pharmaceutique comprenant comme principe actif au moins un composé tel que défini ci-dessus, notamment un composé peptidique.

Elle a notamment pour objet toute composition pharmaceutique comprenant comme principe actif au moins un anticorps et/ou un fragment d'anticorps tel que défini ci-dessus, ainsi que toute composition pharmaceutique comprenant comme principe actif au moins un acide nucléique ou un vecteur tel que défini ci-dessus.

Elle a également pour objet toute composition pharmaceutique comprenant comme principe actif une molécule chimique capable d'augmenter ou de diminuer l'interaction entre la protéine PAP1 et la Parkine.

Par ailleurs, elle a aussi pour objet les compositions pharmaceutiques dans lesquelles les peptides, anticorps, molécules chimiques et séquences nucléotidiques définis ci-dessus sont associés entre-eux ou avec d'autres principes actifs.

Les compositions pharmaceutiques selon l'invention peuvent être utilisées pour moduler l'activité de la protéine Parkine et de ce fait maintenir la survie des neurones dopaminergiques. Plus particulièrement, ces compositions pharmaceutiques sont destinées à moduler l'interaction entre la protéine PAP1 et la Parkine. Il s'agit plus préférentiellement de compositions pharmaceutiques destinées au traitement de maladies du système nerveux central comme par exemple la maladie de Parkinson.

L'invention a encore pour objet l'utilisation des molécules décrites ci-avant pour moduler l'activité de la Parkine ou le typage de maladies du système nerveux central. En particulier, l'invention concerne l'utilisation de ces molécules pour moduler au moins partiellement l'activité de la Parkine.

L'invention concerne également un procédé pour le criblage ou la caractérisation de molécules actives sur la fonction de la parkine, comprenant la sélection de molécules capables de lier la séquence SEQ ID NO :2 ou la séquence SEQ ID NO :4, ou un fragment (ou dérivé) de celles-ci. Le procédé comprend avantageusement la mise en contact, in vitro, de la ou des molécules test avec un polypeptide comprenant la séquence SEQ ID NO :2 ou la séquence SEQ ID NO :4, ou un fragment (ou dérivé) de celles-ci, et la sélection de molécules capables de lier la séquence SEQ ID NO :2 (notamment la région comprise entre les résidus 1 et 344) ou la séquence SEQ ID NO :4. Les molécules testées peuvent être de nature variée (peptide, nucléique, lipide, sucre, etc., ou des mélanges de telles molécules, par exemple des bibliothèques combinatoires, etc.). Comme indiqué ci-avant, les molécules ainsi identifiées peuvent être utilisées pour moduler l'activité de la protéine Parkine, et représentent des agents thérapeutiques potentiels pour le traitement de pathologies neurodégénératives.

D'autres avantages de la présente invention apparaîtront à la lecture des exemples et figure qui suivent, qui doivent être considérés comme illustratifs et non limitatifs.

### LEGENDES DE LA FIGURE :

Figure 1: Représentation du vecteur pLex9-Parkine (135-290)
Figure 2 : Résultats de la première expérience 5'-RACE. 8 clones ont été obtenus. La séquence électronique initiale est indiquée au bas de la figure.
Figure 3 : Résultats de la seconde expérience 5'-RACE. Seulement deux des 8 clones obtenus dans la première expérience ont été validés (clones A12 et D5). La séquence électronique initiale est indiquée au bas de la figure. La séquence complète des ADN et protéines et donnée dans les Séquences 12-15.
Figure 4 : Détail de l'organisation des clones C5 et D4 de la seconde expérience 5'-RACE. La séquence consensus résultante est indiquée en haut de la figure.
Figure 5 : Structure des transcrits isolés à partir de cerveau humain.
Figure 6 : Séquence nucléique et protéique de LY111 (full length) de cerveau humain. Double souligné : cystéines conservées du domaine en doigt de zinc. Gras : Domaine C₂l, Italique : domaine C₂2.
Figure 7 : Séquence nucléique et protéique de LY111 (version courte) de cerveau humain. Double souligné : cystéines conservées du domaine en doigt de zinc. Gras : Domaine C₂l, Italique : domaine C₂2.
Figure 8 : Localisation de la protéine LY111 courte (8b) ou longue (8a) après expression dans les cellules Cos-7.
Figure 9 : Séquence nucléique et protéique de LY111 (version longue) de poumon humain.
Figure 10 : Séquence nucléique et protéique de LY 111 (version courte) de cerveau humain.

### MATERIELS ET TECHNIQUES MIS EN OEUVRE

### 1) Souches de levure:

La souche L40 du genre *S.cerevisiae* (*Mata, his3D200, trp1-901, leu2-3,112, ade2, LYS2:: (lexAop)₄-HIS3, URA3::(lexAop)₈LacZ, GAL4, GAL80*) a été utilisée pour vérifier les interactions protéine-protéine quand l'un des partenaires protéiques est fusionné à la protéine LexA. Cette dernière est capable de reconnaître l'élément de réponse LexA contrôlant l'expression des gènes rapporteurs *LacZ* et His3.

Elle a été cultivée sur les milieux de culture suivants:.
Milieu YPD complet :
-Extrait de levures (10 g/l) (Difco)
-Bactopeptone (20 g/l) (Difco)
-Glucose (20 g/l) (Merck)
Ce milieu a été rendu solide par addition de 20 g/l d'agar (Difco).
Milieu YNB minimum :
- Yeast Nitrogen Base (sans acides aminés) (6,7 g/l) (Difco)
- Glucose (20 g/l) (Merck)
Ce milieu peut être rendu solide par addition de 20 g/l d'agar (Difco). Il peut également être supplémenté en acides aminés et/ou en 3-amino-1,2,4-triazole par addition de milieux CSM [CSM -Leu, -Trp, -His (620 mg/l), CSM -Trp (740 mg/l) ou CSM -Leu, -Trp (640 mg/l)(Bio101)] et/ou de 3-amino-1,2,4-triazole 2,5 mM.

### 2) Souches de bactéries:

La souche TG1 d'*Escherichia coli,* de génotype supE, hsdΔ5, thi, Δ(lac-proAB), F'[tra D36 pro A⁺B⁺ lacI^{q} lacZΔM15], a été employée pour la construction de plasmides, comme moyen d'amplification et d'isolement de plasmides recombinants utilisés. Elle a été cultivée sur le milieu suivant :
Milieu LB:
- NaCl (5 g/l) (Prolabo)
- Bactotryptone (10 g/l) (Difco)
- Extrait de levure (5 g/l) (Difco)
Ce milieu est rendu solide par addition de 15 g/l d'agar (Difco).

L'ampicilline a été utilisée à 100 µg/ml, cet antibiotique sert à sélectionner les bactéries ayant reçu les plasmides portant comme marqueur le gène de résistance à cet antibiotique.

La souche HB101 d'*Escherichia coli* de génotype supE44, aral4, galK2, lacYl, Δ(gpt-proA)62, rpsL20(Str^{r}), xyl-5, mtl-1, recA13, Δ(mcrC-mrr), HsdS⁻(r⁻m⁻) a été employée comme moyen d'amplification et d'isolement de plasmides provenant de la banque d'ADNc de lymphocyte humain. Elle a été cultivée sur

### Milieu M9: -Na2HPO4 (7 g/l) (Prolabo)

- KH2PO4 (3 g/l) (Prolabo)
- NH4Cl (1 g/l) (Prolabo)
- NaCl (0,5 g/l) (Prolabo)
- Glucose (20 g/l) (Sigma)
- MgSO4 (1 mM) (Prolabo)
- Thiamine (0,001 %) (Sigma)
Ce milieu est rendu solide par addition de 15 g/l d'agar (Difco).
De 1a leucine (50 mg/l) (Sigma) et de la proline (50 mg/l) (Sigma) doivent être ajoutées au milieu M9 pour permettre la croissance de la souche HB101.

Lors de la sélection de plasmides provenant de la banque deux hybrides d'ADNc de lymphocyte, la leucine n'a pas été ajoutée au milieu car les plasmides portent un marqueur de sélection Leu2.

### 3) Plasmides :

Le vecteur pLex9 (pBTM116) (Bartel *et al*., 1993) de 5kb homologue au pGBT10 qui contient un site multiple de clonage situé en aval de la séquence codant pour le répresseur bactérien LexA et en amont d'un terminateur pour former une protéine de fusion.

pLex-HaRasVal12, plasmide pLex9, tel que décrit dans la demande WO98/21327, qui contient la séquence codant pour la protéine HaRas mutée en position Val12 connue pour interagir avec la protéine Raf de mammifère (Vojtek *et al*., 1993). Ce plasmide a été utilisé pour tester la spécificité d'interaction de la protéine PAP1 dans la souche L40.

pLex9-cAPP, plasmide pLex9 qui contient la séquence codant pour le domaine cytoplasmique de la protéine APP connue pour interagir avec le domaine PTB2 de FE65. Ce plasmide a été utilisé pour tester la spécificité d'interaction de la protéine PAP1 dans la souche L40.

### 4) Oligonucléotides de synthèse:

| | |
|---|---|
| TTAAGAATTC GGAAGTCCAG CAGGTAG | (SEQ ID N°5) |
| ATTAGGATCC CTACACACAA GGCAGGGAG | (SEQ ID N°6) |

Oligonucléotides qui ont permis d'obtenir le fragment PCR correspondant à la région centrale de la Parkine bordée par les sites *EcoRI* et *BamHI.*

| | |
|---|---|
| GCGTTTGGAA TCACTACAG | (SEQ ID N°7) |
| GGTCTCGGTG TGGCATC | (SEQ ID N°8) |
| CCGCTTGCTT GGAGGAAC | (SEQ ID N°9) |
| CGTATTTCTC CGCCTTGG | (SEQ ID N° 10) |
| AATAGCTCGA GTCAGTGCAG GACAAGAG | (SEQ ID N°11) |

Oligonucléotides qui ont servi à séquencer l'insert correspondant au gène PAP1.

Les oligonucléotides sont synthétisés sur l'appareil Applied System ABI 394-08. Ils sont décrochés de la matrice de synthèse par de l'ammoniac et précipités deux fois par 10 volumes de n-butanol puis repris dans de l'eau. La quantification est effectuée par mesure de la densité optique (1DO₂₆₀ correspond à 30 µg/ml).

### 5) Préparation des ADN plasmidiques.

Les préparations en petite quantité et en grande quantité d'ADN plasmidique ont été effectuées selon les protocoles recommandés par le fabricant Quiagen des kits de purification d'ADN :
- Quiaprep Spin Miniprep kit, ref : 27106
- Quiaprep Plasmid Maxiprep kit, ref : 12163.

### 6) Amplification enzymatique d'ADN par PCR (Polymerase Chain Reaction) :

Les réactions de PCR sont effectuées dans un volume final de 100µl en présence de la matrice d'ADN, de dNTP (0,2 mM), de tampon PCR (Tris-HCl pH 8,5 10 mM, MgCl₂ 1 mM, KCl 5 mM, gélatine 0,01%), de 10-20 pmoles de chacun des oligonucléotides et de 2,5 UI d'Ampli Taq DNA polymérase (Perkin Elmer). Le mélange est recouvert de 2 gouttes d'huile de paraffine pour limiter l'évaporation de l'échantillon. L'appareil utilisé est le "Crocodile II" d'Appligene.

Nous avons utilisé une température de dénaturation de la matrice de 94°C, une température d'hybridation de 52°C et une température d'élongation par l'enzyme à 72°C.

### 7) Les ligatures :

Toutes les réactions de ligation sont effectuées à 37°C pendant une heure dans un volume final de 20 µl en présence de 100 à 200 ng de vecteur, 0,1 à 0,5 µg d'insert, 40 UI d'enzyme T4 DNA ligase (Biolabs) et un tampon de ligation (Tris-HCl 50 mM pH 7,8; MgCl₂ 10 mM; DTT 10 mM; ATP 1 mM). Le témoin négatif est constitué par la ligation du vecteur en l'absence d'insert.

### 8) Tranformation des bactéries :

La tranformation des bactéries par un plasmide est effectuée selon le protocole suivant :10µl du volume de ligation est utilisée pour transformer les bactéries TG1 selon la méthode la méthode de Chung (Chung *et al*., 1989). Après transformation les bactéries sont étalées sur un milieu LB + ampicilline et incubées 16h à 37°C.

### 9) Séparation et extraction des ADN:

La séparation des ADN est réalisée en fonction de leur taille par électrophorèse sur gel d'agarose selon Maniatis (Maniatis *et al*., 1989) : gel d'agarose à 1% (Gibco BRL) dans un tampon TBE (Tris base 90 mM; Borate 90 mM; EDTA 2 mM)

### 10) Séquençage fluorescent des ADN plasmidiques :

La technique de séquençage utilisée est dérivée de la méthode de Sanger (Sanger *et al*., 1977) et adaptée pour le séquençage par fluorescence développé par Applied Biosystems. Le protocole utilisé est celui décrit par les concepteurs du système (Perkin Elmer, 1997).

### 11) Transformation de la levure:

Les plasmides sont introduits dans la levure par une technique classique de transformation de la levure mise au point par Gietz (Gietz *et al*., 1992) et modifiée de la façon suivante :

Dans le cas particulier de la tranformation de la levure par la banque d'ADNc de lymphocyte, la levure utilisée contient le plasmide pLex9-Parkine (135-290) codant pour la partie centrale de la Parkine fusionnée à la protéine LexA. Elle est cultivée dans 200 ml de milieu minimum YNB supplémenté en acides aminés CSM - Trp à 30°C sous agitation jusqu'à une densité de 10⁷ cellules/ml. Pour effectuer la transformation des levures suivant le protocole précédant, la suspension cellulaire a été séparée en 10 tubes de 50µl dans lesquels 5µg de la banque ont été ajoutés. Le choc thermique a été effectué pendant 20 minutes puis les cellules ont été collectées par centrifugation et resuspendues dans 100 ml de milieu YPD pendant 1h à 30°C et dans 100 ml de milieu YNB supplémenté en CSM -Leu, -Trp pendant 3h30 à 30°C. L'efficacité de la transformation est déterminée en étalant différentes dilutions de cellules transformées sur milieu YNB solide supplémenté en CSM -Trp, -Leu. Après une culture à 30°C durant 3 jours les colonies obtenues ont été comptées et le taux de transformation par µg d'ADN de la banque de lymphocyte a été déterminé.

### 12) Isolement de plasmides extrait de levure :

5 ml d'une culture de levure incubée 16h à 30°C sont centrifugés et repris dans 200 µl d'un tampon de lyse (Sorbitol 1M, KH₂PO₄/K₂HPO₄ 0,1M pH7,4, zymolyase 12,5 mg/ml) et incubés 1h à 37°C. Le lysat est ensuite traité selon le protocole recommandé par le fabriquant Quiagen du kit de purification d'ADN, Quiaprep Spin Miniprep kit, ref 27106.

### 13) Test d'activité de la β-galactosidase :

Une feuille de nitrocellulose est préalablement déposée sur la boite de Pétri contenant les clones de levures individualisés. Cette feuille est ensuite plongée dans de l'azote liquide pendant 30 secondes afin de faire éclater les levures et de libérer ainsi l'activité β-galactosidase. Après décongélation, la feuille de nitrocellulose est déposée, colonies vers le haut, dans une autre boite de Pétri contenant un papier Whatman préalablement imbibé de 1,5 ml de solution PBS (Na₂HPO₄ 60 mM, NaH₂PO₄ 40 mM, KCl 10 mM, MgSO₄ 1 mM, pH7) contenant 15 µl de X-Gal (5-bromo-4-chloro-3-indoyl-β-D-galactoside) à 40 mg/ml de N,N-diméthylformamide. La boite est ensuite placée dans une étuve à 37°C. Le test est dit positif lorsque les colonies virent au bleu sur la membrane au bout de 12 heures.

### EXEMPLE 1: CONSTRUCTION D'UN VECTEUR PERMETTANT L'EXPRESSION D'UNE PROTEINE DE FUSION ENTRE LA PARTIE CENTRALE DE LA PARKINE ET LE REPRESSEUR BACTERIEN LEXA.

Le criblage d'une banque utilisant le système double hybride nécessite que la région centrale de la Parkine soit fusionnée à une protéine liant l'ADN comme le répresseur bactérien LexA. L'expression de cette protéine de fusion est réalisée grâce au vecteur pLex9 (cf. matériels et méthodes), dans lequel a été introduite, dans le même cadre de lecture que la séquence correspondant à la protéine LexA, la séquence codant pour la région centrale de la Parkine figurant dans la séquence présentée en SEQ ID N°3 ou 4.

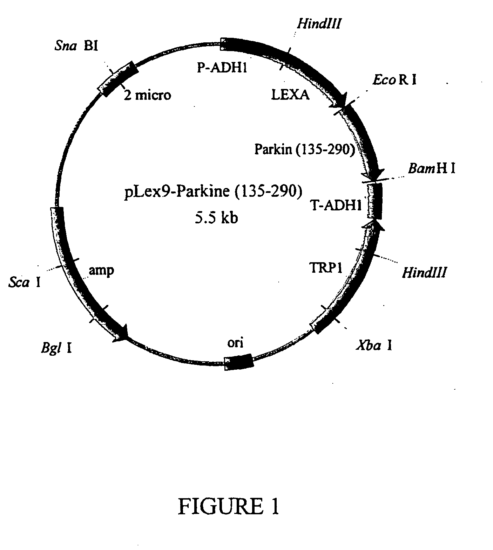

Le fragment d'ADN de 468 pdb correspondant aux 156 acides aminés de la région centrale de la Parkine qui commence à l'acide aminé 135 a été obtenu par PCR à partir des oligonucléotides (SEQ ID N°5 et N°6) qui ont également permis d'introduire le site *EcoRI* à l'extrémité 5' et un codon stop et un site *BamHI* à l'extrémité 3'. Le fragment de PCR a été introduit entre les sites *EcoRI* et *BamHI* du multisite de clonage du plasmide pLex9 en aval de la séquence codant pour la protéine LexA pour donner le vecteur pLex9-Parkine (135-290) (Fig.1).

La construction a été vérifiée par séquençage de l'ADN. Cette vérification a permis de montrer que ce fragment ne présentait pas de mutations générées au cours de la réaction de PCR et qu'il était fusionné dans la même phase ouverte de lecture que celle du fragment correspondant à LexA.

### EXEMPLE 2: CRIBLAGE D'UNE BANQUE DE FUSION DE LYMPHOCYTE.

Nous avons utilisé la méthode du Double-Hybride (Fields et Song, 1989).

Le criblage d'une banque de fusion permet d'identifier des clones produisant des protéines fusionnées au domaine transactivateur de GAL4, pouvant interagir avec la protéine d'intérêt décrite dans l'exemple 1 (région centrale de la parkine). Cette interaction permet de reconstituer un transactivateur qui va alors être capable d'induire l'expression des gènes rapporteurs His3 et *LacZ* dans la souche L40.

Pour effectuer ce criblage nous avons choisi une banque de fusion réalisée à partir d'ADNc provenant de lymphocytes humains périphériques fournie par Richard Benarous (Peytavi *et al*., 1999). Des levures ont été transformées par la banque de lymphocytes et des clones positifs ont été sélectionnés comme décrit ci-après.

Lors du criblage il est nécessaire de préserver la probabilité que chaque plasmide indépendant de la banque de fusion soit présent dans au moins une levure en même temps que le plasmide pLex9-Parkine (135-290). Pour préserver cette probabilité il est important d'avoir une bonne efficacité de transformation de la levure. Pour cela nous avons choisi un protocole de transformation de la levure donnant une efficacité de 2,6 10⁵ cellules transformées par µg d'ADN. De plus comme la cotransformation de la levure par deux plasmides différents réduit cette efficacité, nous avons préféré utiliser une levure préalablement transformée par le plasmide pLex9-Parkine (135-290). Cette souche L40 pLex9-Parkine (135-290) de phénotype His-, Lys-, Leu-, Adé- a été transformée par 50µg d'ADN plasmidique de la banque de fusion. Cette quantité d'ADN nous a permis d'obtenir après estimation 1,3 10⁷ cellules transformées, ce qui correspond à un nombre légèrement supérieur au nombre de plasmides indépendants qui constituent la banque. D'après ce résultat nous pouvons penser que la quasi totalité des plasmides de la banque a servi à transformer les levures. La sélection des cellules transformées, capables de reconstituer un transactivateur fonctionnel, a été faite sur un milieu YNB supplémenté avec 2,5 mM 3-amino-1,2,4-triazole et 620 mg/l de CSM (Bio101) ne contenant pas d'histidine de leucine et de tryptophane.

A l'issue de cette sélection, de nombreux clones avec un phénotype His+ ont été obtenus. Sur ces transformants un test d'activité β-galactosidase a été effectué afin de valider par l'expression de l'autre gène rapporteur, *LacZ,* ce nombre de clones obtenus. 115 clones présentaient le double phénotype His+, β-Gal+ pouvant correspondre à une interaction protéine-protéine.

### EXEMPLE 3: ISOLEMENT DES PLASMIDES DE LA BANQUE DANS LES CLONES SELECTIONNES.

Afin d'identifier les protéines pouvant interagir avec la région centrale de la Parkine, les plasmides de la banque de fusion contenus dans les levures sélectionnées lors du criblage double hybride ont été extraits. Pour pouvoir en obtenir en grande quantité, cet isolement nécessite au préalable une transformation d'*E.coli* par un extrait d'ADN des souches de levures positives. Comme le plasmide de la banque contenu dans cet extrait est un plasmide navette levure/*E.coli* il peut facilement se répliquer chez la bactérie. Le plasmide de la banque a été sélectionné par complémentation de la bactérie HB101 auxotrophe pour la leucine sur milieu dépourvu de leucine.

Les ADN plasmidiques des colonies bactériennes obtenues après transformation par des extraits d'ADN de levures ont été analysés par digestion avec des enzymes de restriction et séparation des fragments d'ADN sur gel d'agarose. Parmi les 115 clones analysés, un clone contenant un plasmide de la banque présentant un profil différent des autres a été obtenu. Ce plasmide, appelé pGAD-Ly1111b, a été plus précisément étudié.

### EXEMPLE 4: DETERMINATION DE LA SEQUENCE DE L'INSERT CONTENU DANS LE PLASMIDE IDENTIFIE.

Le séquençage de l'insert contenu dans le plasmide identifié a été réalisé dans un premier temps à partir de l'oligonucléotide SEQ ID N°7 complémentaire de la séquence de GAL4TA à proximité du site *EcoRI* d'insertion de la banque de cDNA de lymphocytes. Puis dans un deuxième temps à partir des oligonucléotides SEQ ID N°8 à SEQ ID N°11, correspondant à la séquence de l'insert obtenue au cours de la progression du séquençage. La séquence obtenue est présentée sur la séquence SEQ ID NO1. La protéine ainsi identifiée a été désignée PAP1 (Parkin-Associated Protein 1).

La comparaison de la séquence de cet insert avec les séquences contenues dans les banques de données GENBank et EMBL (European Molecular Biology Lab) a montré une homologie de 25%, au niveau protéique, avec différents membres de la famille des Synaptotagmines. Les Synaptotagmines font partie d'une famille de protéines membranaires codées par au moins onze gènes différents exprimés dans le cerveau et autres tissus. Elles contiennent un domaine transmembranaire unique et deux domaines régulés par le calcium appelés C₂. C'est dans ce domaine que l'on trouve l'homologie entre les Synaptotagmines et la protéine PAP1. Aucune autre homologie significative n'a été observée.

### EXEMPLE 5: ANALYSE DE LA SPECIFICITE D'INTERACTION ENTRE LA REGION CENTRALE DE LA PARKINE ET LA PROTEINE PAP1.

Afin de déterminer la spécificité d'interaction entre le fragment correspondant à la protéine PAP1 et la région centrale de la Parkine, un test d'interaction spécifique deux hybrides avec d'autres protéines non relevantes a été réalisé. Pour réaliser ce test nous avons transformé la souche L40 par les plasmides de contrôle plex9-cAPP ou pLex9-HaRasVal12 à la place du plasmide pLex9-Parkin (135-290) codant respectivement pour le domaine cytoplasmique de l'APP ou la protéine HaRasVal12 fusionnés au domaine le liaison à l'ADN de LexA et par le plasmide isolé lors du criblage de la banque deux hybrides. Un test d'activité β-Gal sur les cellules transformées par les différents plasmides a été effectué afin de déterminer une interaction protéine-protéine. D'après le résultat du test, seules les levures transformées par le plasmide isolé lors du criblage de la banque deux hybrides et par le plasmide pLex9-Parkine (135-290) présentaient une activité β-Gal+ montrant ainsi une interaction entre la région centrale de la Parkine et la protéine PAP1. Cette interaction s'avère donc être spécifique puisque ce fragment de PAP1 ne semble pas interagir avec les protéines cAPP ou HaRasVal12.

Ces résultats montrent donc l'existence d'une protéine nouvelle, désignée PAP1, capable d'interagir de manière spécifique avec la Parkine. Cette protéine, apparentée aux synaptotagmines, ne présente aucune homologie significative avec des protéines connues, et peut être utilisée dans des applications thérapeutiques ou diagnostiques, pour la production d'anticorps, de sondes ou peptides ou encore pour le criblage de molécules actives.

### EXEMPLE 6 : CLONAGE DU GENE PAP1 A PARTIR D'UNE BANQUE D'ADN DE POUMON HUMAIN

Dans le but d'identifier la séquence complète du gène PAP1 humain et de caractériser l'existence de formes variantes, deux approches d'élongation électronique ont été réalisées à partir de la séquence SEQ ID NO : 1. Deux séquences électroniques ont ainsi été obtenues, de 1644 pb et 1646 pb respectivement, comportant une élongation de 330 pb par rapport à la séquence SEQ ID NO :1. Néanmoins, l'analyse de ces séquences a montré des différences dans la région consensus, apparentes après traduction. Ainsi, une ORF de 420aa est obtenue dans un cas, et une ORF de 230aa avec l'autre séquence. La séquence protéique obtenue a été comparée avec les séquences connues, et a révélé une homologie de 24% sur les 293 acides aminés recouvrant avec la synaptogamine humaine 1 (p65)(p21579). La fonction de la synaptogamine I meut être un rôle régulateur dans les interactions membranaires au cours du trafic de vésicules synaptiques dans la zone de la synapse. Elle lie les phospholipides acides avec une certaine spécificité. En outre, une interaction dépendante du calcium entre la synaptogamine et les récepteurs de protéine kinase C activée a été rapportée. La synaptogamine peut également lier trois autres protéines que sont la neurexine, la syntaxine et ap2. Compte tenu de la disparition brutale et précoce de toute homologie entre les séquences identifiées et la famille des synaptogamines, il est possible que la séquence identifiée présentent une délétion par rapport à la séquence naturelle. Pour vérifier cette hypothèse et valider les séquences une expérience de RT-PCR et séquençage a été réalisée en utilisant la séquence de 1644 pb. La séquence obtenir comporte une ORF de 420aa présentant une homologie de même ordre avec les synaptogamines.

Pour tenter d'obtenir une séquence plus grande, et vérifier si la séquence obtenue pouvait correspondre à une forme d'épissage, une expérience d'élongation par 5'-RACE a été initiée à partir de la région 3' de la séquence validée, au moyen des oligos L1 et L2 sur une préparation d'ADNc de poumon humain.

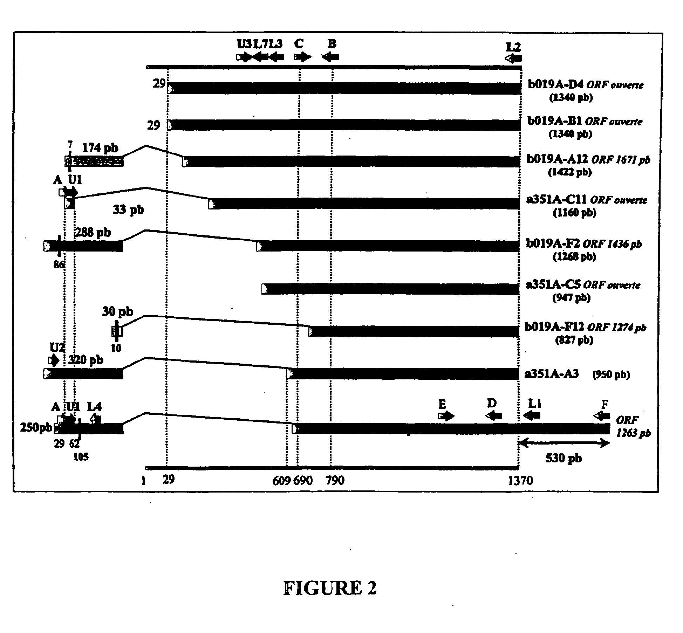

Les résultats obtenus sont présentés sur la figure 2 et montrent l'identification de 8 clones correspondant à 6 extrémités 5' terminales différentes. Trois d'entre elles contiennent un codon STOP qui interrompt l'ORF (clones A12, F2, F12) et le clone A3 ne contient aucune ORF. La présence de différents transcrits a été confirmée par RT-PCR et nested RT-PCR (Table 1).

**Table 1**

| RT-PCR | Primaire | PCR Secondaire U3-L3 | PCR Secondaire U1-L4 | PCR Secondaire C-B |
|---|---|---|---|---|
| U3-L3 | 170 | | | |
| A-L4 | 153 | | + | |
| A-L3 | Smear | + | | |
| U1-L4 | 130 | | | |
| U1-L3 | Smear | + | | |
| U1-B | 415 | | | + |
| U2-B | 515 | | | + |
| Taille attendue | | 170 | 130 | 120 |

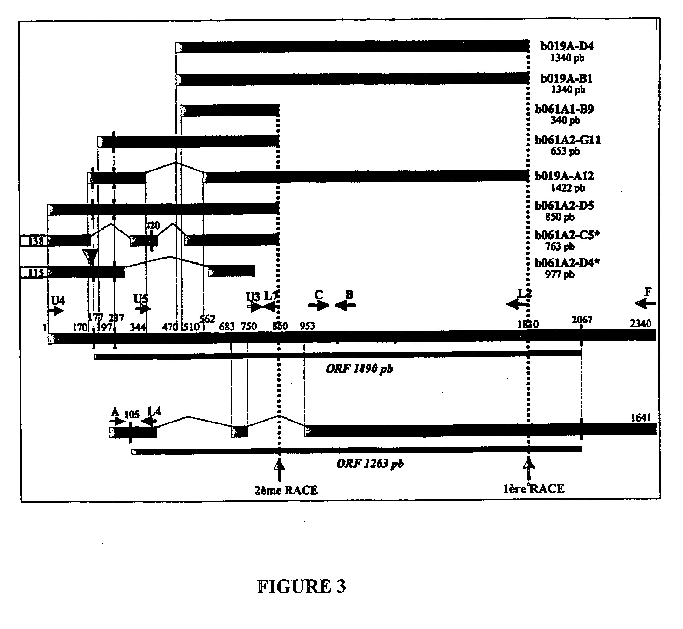

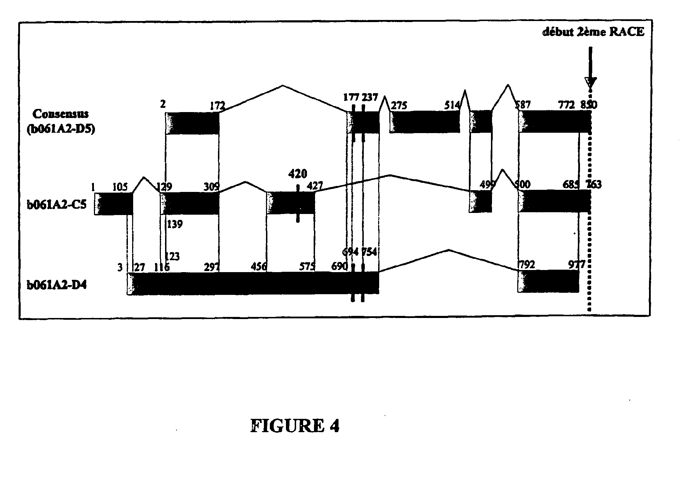

Les couples d'amorces U3-L3 et C-B sont spécifiques du fragment commun de la séquence, les oligo A et U1 sont spécifiques de la séquence initiale et du clone C11, l'oligo L4 est spécifique de la séquence initiale et l'amorce U2 est spécifique du clone A3. Une seconde 5'-RACE a été réalisée avec les oligos L3 et L7 localisés dans la région commune des différents clones (Figure 2). Les résultats obtenus sont présentés dans les Figures 3 et 4. La présence de différents transcrits a été confirmée par RT-PCR et nested RT-PCR (Table 2).

**Table 2**

| RT-PCR | Résultat | PCR Secondaire C-B | PCR Secondaire U3-B | PCR Secondaire U5-L7 |
|---|---|---|---|---|
| U4-F | Smear | + | + | + |
| U5-F | Smear | + | + | + |
| U3-F | 1550 pb | + | + | |
| Taille attendue (pb) | | 120 | 385 | 530 |

La séquence des amorces et oligonucléotides est donnée dans les Tableaux 3 et 4 (SEQ ID NO : 16-37).

**Tableau 3**

| | | | | SEQ ID |
|---|---|---|---|---|
| LY111_U4 | CCAGTTCTGCCTGTTCATC | 23 | à 41 | 16 |
| LY111_U5 | TTCAAAACACAGAGGAGGAG | 319 | à 338 | 17 |
| LY111_U3 | GAATTTGGTCAGTTTAGAGG | 759 | à 778 | 18 |
| LY111_L7 | TTCTGGGATTTGGAGAGCTTTTTCAC | 851 | à 825 | 19 |
| LY111_L6 | TCTGTCTGTCCCACACACTGCC | 914 | à 892 | 20 |
| LY111_L3 | GACTGGCTCCGTCTCTCTG | 928 | à 910 | 21 |
| LY111_C | AAGCAACAGAATCTCCCATCC | 1029 | à 1049 | 22 |
| LY111_B | GCATTGTCAAAATTGCCCATC | 1147 | à 1127 | 23 |
| LY111_E | AGGCGGAGAAATACGAAGAC | 1543 | à 1562 | 24 |
| LY111_D | GCAGAGTGAGACAGCCCTTAAC | 1767 | à 1746 | 25 |
| Ly111_L2 | CTTCCTCAGGACTGGCGACTTCAG | 1811 | à 1782 | 26 |
| Ly111_L1 | CAAGCGGTCGTTCATTCCAAAGAG | 1934 | à 1913 | 27 |
| LY111_F | AAGAGGAGATAACCCACCAGAG | 2288 | à 2269 | 28 |

**Tableau 4**

| | | | | |
|---|---|---|---|---|
| LY111_A | TCGTAGAGCAGCAGGTCCAAG | 14 | à 34 | 46 |
| LY111_U1 | AGGGCTGCTGGCTATTTTTC | 36 | à 55 | 29 |
| LY111_L4 | TAAGAAATGGGTTGTGAAC | 148 | à 166 | 30 |
| LY111_C | AAGCAACAGAATCTCCCATCC | 1029 | à 1049 | 31 |
| LY111_B | GCATTGTCAAAATTGCCCATC | 1147 | à 1127 | 32 |
| LY111_E | AGGCGGAGAAATACGAAGAC | 1543 | à 1562 | 33 |
| LY111_D | GCAGAGTGAGACAGCCCTTAAC | 1767 | à 1746 | 34 |
| Ly111_L2 | CTTCCTCAGGACTGGCGACTTCAG | 1811 | à 1782 | 35 |
| Ly111_L1 | CAAGCGGTCGTTCATTCCAAAGAG | 1934 | à 1913 | 36 |
| LY111_F | AAGAGGAGATAACCCACCAGAG | 2288 | à 2269 | 37 |

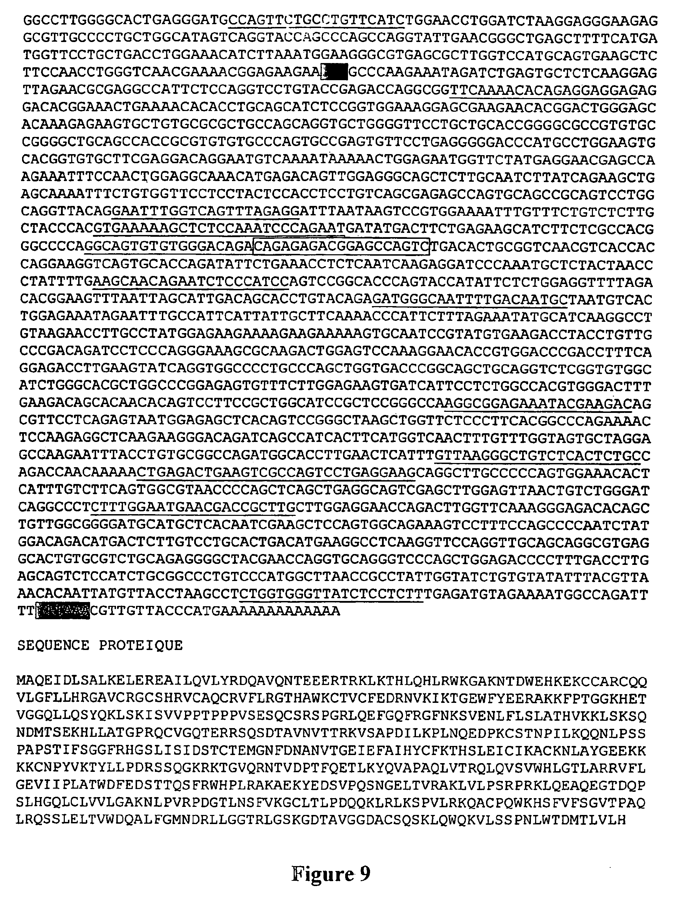

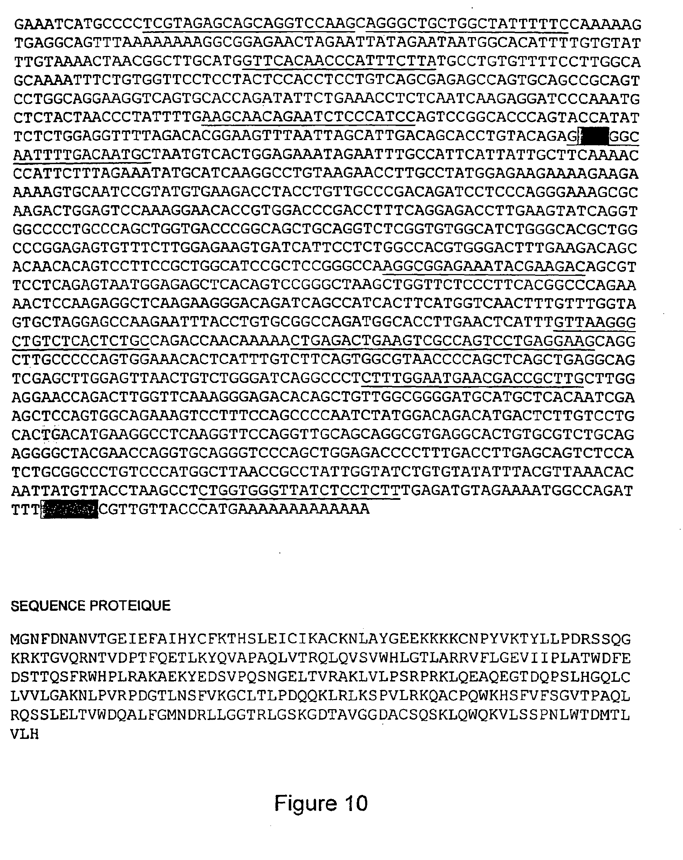

L'ensemble de ces résultats permet de valider la séquence consensus correspondant à l'isoforme longue (Figure 9, SEQ ID N0 :12 et 13) et à l'isoforme courte (Figure 10, SEQ ID NO :14 et 15) de la protéine PAP1 identifiée à partie de poumon humain. Cette protéine est également désignée dans la suite des exemples par le terme LY111. L'isoforme long est codé par une ORF de 1833 pb localisée aux résidus 237-2069 de SEQ ID NO :12, et comprend 610 acides aminés. Le signal de polyadénylation est localisé à partir du nucléotide 2315. L'isoforme court est codé par une ORF de 942 pb localisée aux résidus 429-1370 de SEQ ID NO :14, et comprend 313 acides aminés. Le signal de polyadénylation est localisé à partir du nucléotide 1616.

Des expériences de Northern blot ont ensuite été réalisées sur différents tissus humains avec des sondes (amplimer CD et E-F) et ont permis de révéler un transcrit de 6 kb dans le muscle, un transcrit dans le coeur (3kb), ainsi qu'un transcrit de 6 kb dans le foie foetal. L'Exemple 7 décrit par ailleurs le clonage d'un transcrit dans le cerveau foetal humain.
Différentes études d'homologies ont été effectuées dans différentes bases de données protéiques, dont les résultats sont présentés dans le tableau 5 suivant.

**Tableau 5**

| Banque | Homologie |
|---|---|
| Genpept116 | G5926736 (AB025258) granuphilin-a Identité : 31 % (215/679), Homologie (POS) : 46% (322/679) |
| | G5926738 (AB025259) granuphilin-b Identité : 31 % (150/479), Homologie (POS) : 47% (230/479) |
| | G1235722 (D70830) Doc2 beta (homo sapiens) Identité : 25% (74/292), Homologie (POS) : 43% (127/292) |
| | G289718 (L 15302) Synaptogamin-I Identité : 26% (77/293), Homologie (POS) : 45% (133/293) |
| Swissprot | SP :SYT1_CAEEL Synaptogamin I Identité : 26% (77/293), Homologie (POS) : 45% (133/293) |
| | SP :SYT2_MOUSE Synaptogamin II Identité : 24% (72/293), Homologie (POS) : 44% (131/293) |

### EXEMPLE 7 : CLONAGE DE DEUX TRANSCRITS FULL-LENGTH DE PAP1 (LY111B) A PARTIR D'ADN COMPLEMENTAIRE DE CERVEAU FOETAL HUMAIN

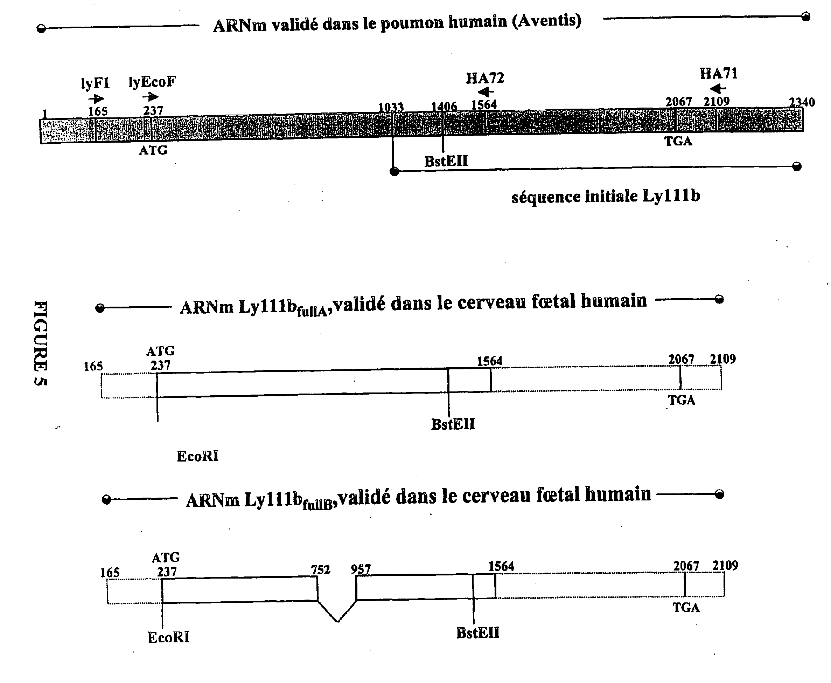

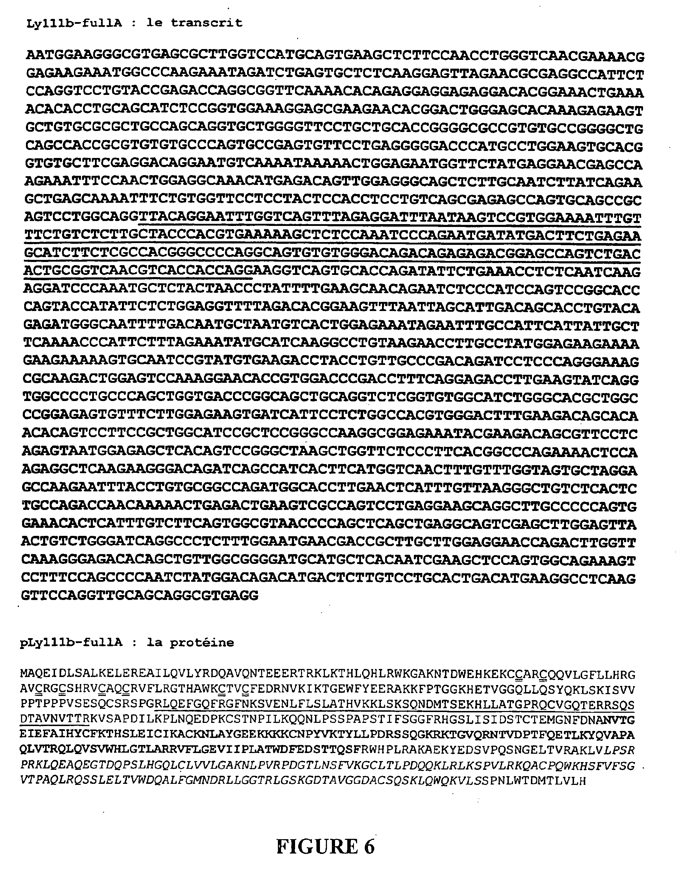

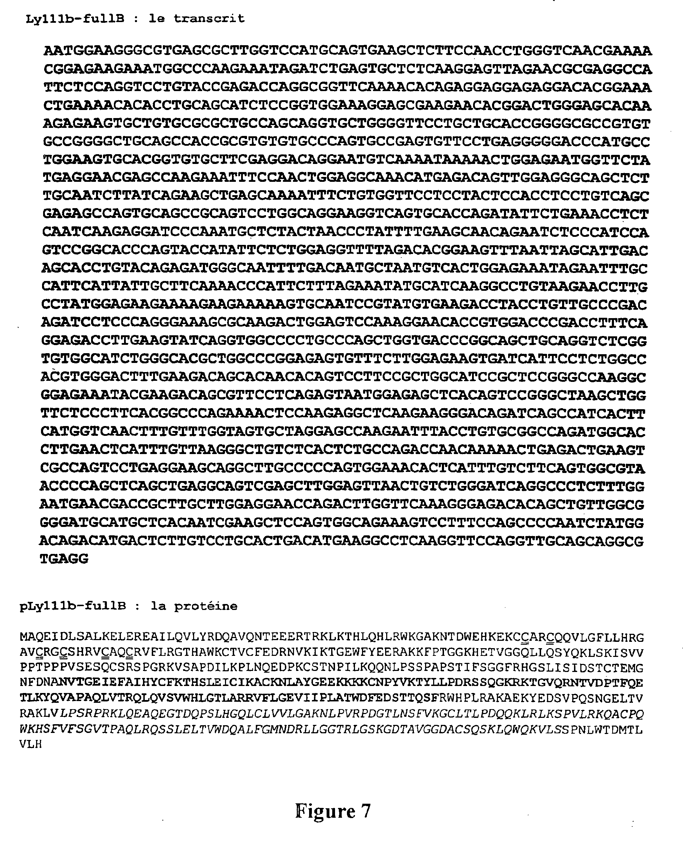

Afin de confirmer la présence d'un transcrit Ly111b *full-length* (« complet ») dans le cerveau humain, une PCR a été effectuée à partir d'ADN complémentaire issu de cerveau foetal humain (Marathon Ready cDNA, Clontech), en utilisant comme amorces les oligonucléotides LyF1 (AAT GGA AGG GCG TGA CGC, figure 5, SEQ ID NO : 38) et HA71 (CCT CAC GCC TGC TGC AAC CTG, SEQ ID NO : 39). Un fragment d'ADN faiblement représenté d'environ deux kilobases a été amplifié. Le produit de cette première PCR a servi de matrice pour une PCR *nested*, effectuée avec les oligonucléotides LyEcoF (GCACGAATTC ATG GCC CAA GAA ATA GAT CTG, SEQ ID NO : 40) et HA72 (CTG TCT TCG TAT TTC TCC GCC TTG, SEQ ID NO : 41). Les produits amplifiés ont été digérés avec les enzymes de restriction EcoRI (intégré dans l'oligonucléotide LyEcoF) et BstEII (figure 5) et insérés dans le vecteur d'expression pcDNA3, puis leur séquence a été déterminée. L'analyse de la séquence des clones obtenus a révélé la présence de deux transcrits Ly111b potentiels *full-length* dans le cerveau foetal humain (figure 5). Le premier de ces transcrits (Ly111b_{fullA}) correspond à l'ARNm identifié dans le poumon humain (Exemple 6) et code une protéine de 609 acides aminés (pLy111b_{fullA}; figures 5, 6, SEQ ID NO : 42-43). Le deuxième (Ly111b_{fullB}) représente vraisemblablement un produit d'épissage alternatif d'un ARNm primaire commun. Dans ce transcrit, identique à Ly111b_{fullA}, la séquence entre les nucléotides 752 et 956 de la séquence validée dans le poumon humain est absente (SEQ ID NO : 42). Ly111b_{fullB} code ainsi une protéine de 541 acides aminées (pLy111b_{fullB}) identique à pLy111_{fullA}, dans laquelle cependant le domaine inclus entre les acides aminés 172 et 240 (figures 5, 7, SEQ ID No : 44-45) vient à manquer. Les deux protéines pLy111b_{fullA/fullB} intègrent le domaine d'interaction avec le fragment de la Parkine comprenant les acides aminés 135 à 290, identifié chez la levure (séquence initiale Ly111b, figure 5), et peuvent donc théoriquement maintenir cette interaction.

### Les protéines pLy111b_{fullA/fullB} appartiennent à la famille RIM/Rabphiline

pLy111b_{fullA/fullB} présentent une homologie avec les protéines de la famille RIM/rabphiline (Wang Y, Sugita S & Südhof TG. The RIM/NIM family of neuronal C2 domain proteins. J Biol Chem (2000) 275, 20033-20044) et en particulier avec les granulophilines (Wang Jie, Takeuchi T, Yokota H & Izumi T. Novel Rabphilin-3-like protein associates with insulin-containing granules in pancreatic beta calls. J Biol Chem (1999) 274, 28542-28548). Elles se caractérisent par la présence d'un domaine *zinc finger* dans la partie N-terminale et de deux domaines C₂ dans la partie C-terminale (Figures 6 et 7). Le domaine *zinc finger* des protéines de la famille RIM/rabphiline a été impliqué dans l'interaction avec les protéines Rab. Ces dernières, des protéines qui fixent le GTP, sont des composantes essentielles de la machinerie du trafique membranaire dans les cellules eucaryotes. D'autre part, il a été décrit que les domaines C₂ des protéines de la famille RIM/rabphiline peuvent lier des membranes à travers l'interaction avec des phospholipides.

### Expression des protéines ply111b_{fullA/fullB} dans les cellules de la lignée cos-7 : co-localisation avec la Parkine

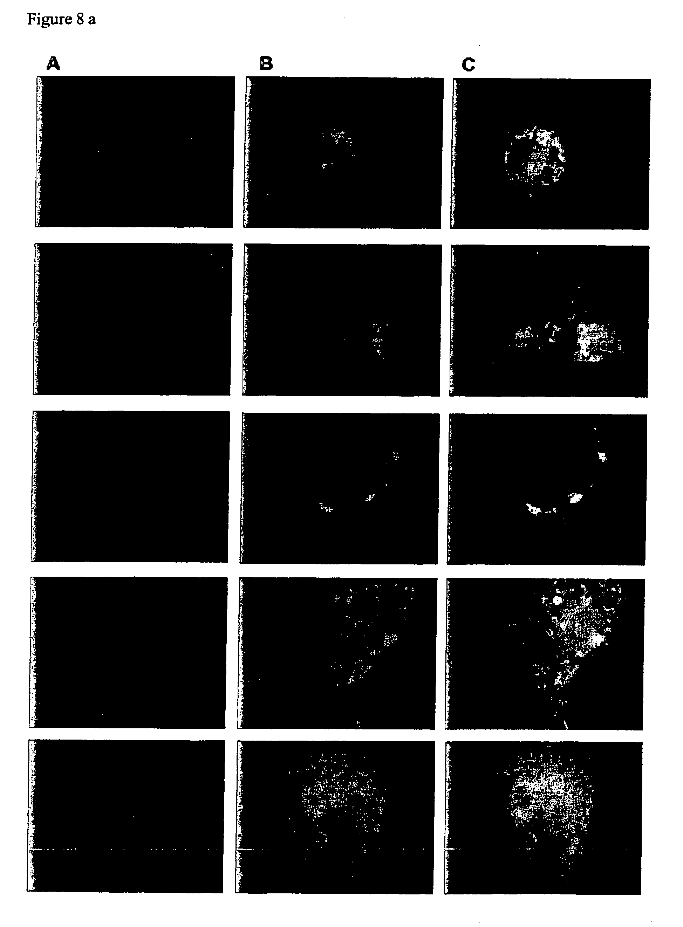

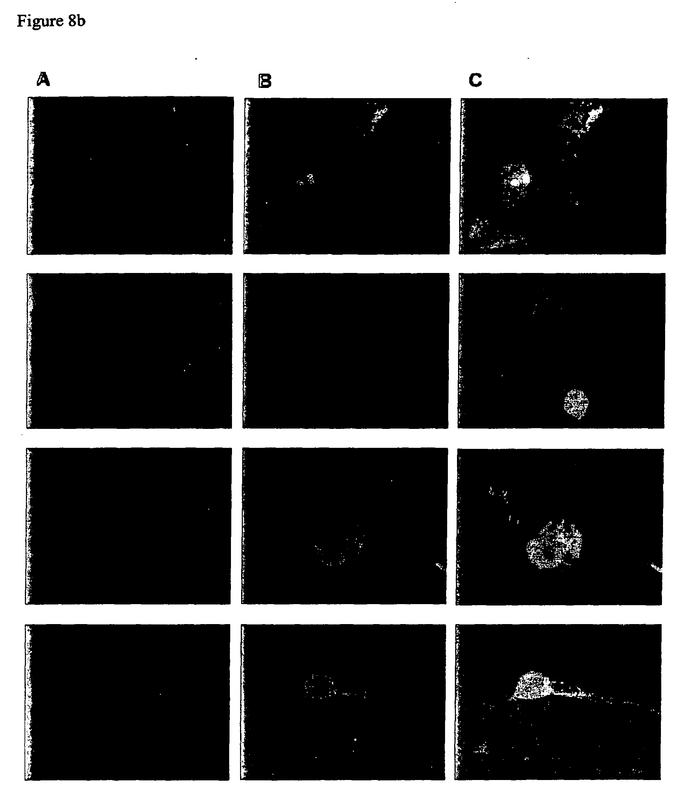

La séquence codante des transcrits Ly111b_{fullA/B} a été insérée dans le vecteur d'expression eucaryote pcDNA3 en phase avec la séquence codant un épitope myc N-terminal (pcDNA3-mycLy111b_{fullA/B}). Des cellules de la lignée cos-7 transfectées à l'aide de ces vecteurs produisent des protéines d'un poids moléculaire apparent d'environ 67 kDa (pcDNA3-mycLy111b_{fullA}) et 60 kDa (pcDNA3-mycLy111b_{fullB}), correspondant au poids moléculaire attendu. Ces protéines, mises en évidence par immunomarquage à l'aide d'un anticorps dirigé contre l'épitope N-terminal myc, se distribuent de manière non homogène, ponctuée, dans le cytoplasme, les prolongements et parfois le noyau des cellules de la lignée cos-7 (figure 8a, b, colonne A). Lorsqu'elles sont sur-exprimées avec la Parkine, révélée à l'aide de l'anticorps anti-Parkine Asp5 dans les cellules de la lignée cos-7 (figure 8a, b, colonne B), une distribution similaire est une co-localisation de ces protéines peut être observée (figure 8a, b, colonne C).

### REFERENCES BIBLIOGRAPHIQUES

Abbas, N. et al. (1999). A wide variety of mutations in the parkin gene are responsible for autosomal recessive parkinsonism in Europe. Hum Mol Genet. 8, 567-574.
**Bartel,** P.L. *et al*. (1993). D.A Hartley Ed, Oxford University press, 153
Chung, CT. et al. (1989). One-step preparation of competent Escherichia coli: transformation andstorage of bacterial cells in the same solution. Proc. Natl. Acad. Sci. USA. 86, 2172-2175.
Fields, S. and Song, O. (1989). A novel genetic system to detect protein-protein interactions. Nature. 340, 245-246.
Gietz, RD. et al. (1992). Improved method for high efficiency transformation of intact yeast cells. Nucleic Acids Res., 20, 1425.
Hattori, N. et al. (1998). Molecular genetic analysis of a novel Parkin gene in Japanese families with autosomal recessive juvenile parkinsonism: evidence for variable homozygous deletions in the Parkin gene in affected individuals. Ann Neurol. 6, 935-941.
Kahn, A. et al., (1991) Thérapie génique : espoirs et limites. Médecine et Sciences. 7, 705-714.
Kitada, T. et al. (1998). Mutations in the parkin gene cause autosomal recessive juvenile parkinsonism. Nature. 392, 605-608.
Leroy, E. et al. (1998). The ubiquitin pathway in Parkinson's disease. Nature. 395, 451-452.
Lewy, F.H. (1912). in Handbuch der Neurologie (Lewandowski, M., ed) pp 920 - 933, Springer, Berlin
Lucking, C. et al. (1998). Homozygous deletions in parkin gene in European and North African families with autosomal recessive juvenile parkinsonism. Lancet. 352, 1355-1356.
Maniatis, T. et al. (1989). Molecular cloning, scond edition. Cold Spring Harbor Laboratory. Cold Spring Harbor, N.Y.
Morett, E. (1999). A novel transactivation domain in parkin. Trends Biochem Sci. 24, 229-231.
Peytavi, R. et al. (1999). HEED, the product of the human homolog of the murine eed gene, binds to the matrix protein of HIV-1. J. Biol. Chem. 274, 1635-1645.
Polymeropoulos, M.H. et al. (1997). Mutation in the alpha-synuclein gene identified in families with Parkinson's disease. Science. 276, 2045-2047.
Sanger, F. et al. (1977). DNA sequencing with chain terminating inhibitors. Proc. Natl. Acad. Sci. USA. 74, 5463-5467.
Shimura, H. et al. (1999). Immunohistochemical and subcellular localization of Parkin protein: absence of protein in autosomal recessive juvenile parkinsonism patients. Ann Neurol. 45, 668-672.
Sunada, Y. et al. (1998). Differential expression of the parkin gene in the human brain and peripheral leukocytes. Neurosci Lett. 254, 180-182.
Vojtek, AB. et al., (1993). Mammalian Ras interacts directly with the serine/threonine kinase Raf. Cell. 74, 205-214.

## Revendications

1. Polypeptide intéragissant avec la Parkine présentant sur toute sa longueur une identité d'au moins 95% avec la séquence SEQ ID NO :2.

2. Polypeptide présentant l'une des séquences SEQ ID NO :13 et SEQ ID NO : 43.

3. Polypeptide présentant l'une des séquences SEQ ID NO :15 et SEQ ID NO :45.

4. Acide nucléique codant pour un polypeptide selon l'une des revendications 1 à 3.

5. Acide nucléique selon la revendication 4. **caractérisé en ce qu'**il présents une identité d'au moins 95% avec la séquence SEQ ID NO :1.

6. Acide nucléique selon la revendication 4, **caractérisé en ce qu'**il présente l'une des séquences SEQ ID NO :12 et SEQ ID NO : 42.

7. Acide nucléique selon la revendication 4 présentant l'une des séquences SEQ ID NO :14 et SEQ ID NO :44.

8. Vecteur comprenant un acide nucléique selon l'une des revendications 4 à 7.

9. Virus recombinant défectif comprenant un acide nucléique selon l'une des revendications 4 à 7.

10. Composition pharmaceutique comprenant au moins un polypeptide selon l'une des revendications 1 à 3.

11. Composition pharmaceutique comprenant au moins un acide nucléique selon l'une des revendications 4 à 7 ou un vecteur selon l'une des revendications 8 ou 9.

12. Composition selon l'une des revendications 10 or 11 destinée au traitement de pathologies neurodégénératives.

13. Procédé pour le criblage ou la caractérisation de molécules destinées au traitement de pathologies neurodégénératives, comprenant une étape de sélection de molécules capables de lier la séquence SEQ ID NO : 2, ou un fragment de celle-ci.

14. Procédé pour le criblage ou la caractérisation de molécules destinées au traitement de pathologies neurodégénératives, comprenant une étape de sélection de molécules capables de lier une séquence choisie parmi les séquences SEQ ID NO : 13, SEQ ID NO : 15, SEQ ID NO : 43 et SEQ ID NO : 45 ou un fragment de celles-ci.

15. Procédé de production d'un polypeptide selon l'une des revendications 1 à 3, comprenant la culture d'une cellule contenant un acide nucléique selon l'une des revendications 4 à 7 ou un vecteur selon la revendication 8 ou 9, dans des conditions d'expression dudit acide nucléique, et la récupération du composé peptidique produit.

16. Cellule contenant un acide nucléique selon l'une des revendications 4 à 7 ou un vecteur selon la revendication 8 ou 9.

17. Mammifère non-humain comprenant dans ses cellules un acide nucléique selon l'une des revendications 4 à 7.

18. Anticorps, fragments ou dèrivés l'anticorps dirigés contre un polypeptide selon l'une des revendications 1 à 3.

## Claims

1. Polypeptide which interacts with parkin, exhibiting, over its entire length, at least 95% identity with the sequence SEQ ID NO: 2.

2. Polypeptide having either of the sequences SEQ ID NO: 13 and SEQ ID NO: 43.

3. Polypeptide having either of the sequences SEQ ID NO: 15 and SEQ ID NO: 45.

4. Nucleic acid encoding a polypeptide according to one of Claims 1 to 3.

5. Nucleic acid according to Claim 4, **characterized in that** it exhibits at least 95% identity with the sequence SEQ ID NO. 1.

6. Nucleic acid according to Claim 4, **characterized in that** it has either of the sequences SEQ ID NO: 12 and SEQ ID NO: 42.

7. Nucleic acid according to Claim 4, having either of the sequences SEQ ID NO: 14 and SEQ ID NO: 44.

8. Vector comprising a nucleic acid according to one of Claims 4 to 7.

9. Defective recombinant virus comprising a nucleic acid according to one of Claims 4 to 7.

10. Pharmaceutical composition comprising at least one polypeptide according to one of Claims 1 to 3.

11. Pharmaceutical composition comprising at least one nucleic acid according to one of Claims 4 to 7, or one vector according to either of Claims 8 and 9.

12. Composition according to either of Claims 10 and 11, intended for treating neurodegenerative pathologies.

13. Method for screening or for characterizing molecules intended for treating neurodegenerative pathologies, which comprises a step of selecting molecules which are capable of binding the sequence SEQ ID NO. 2, or a fragment of this sequence.

14. Method for screening or for characterizing molecules intended for treating neurodegenerative pathologies, which comprises a step of selecting molecules which are capable of binding a sequence chosen from the sequences SEQ ID NO: 13, SEQ ID NO: 15, SEQ ID NO: 43 and SEQ ID NO: 45, or a fragment of these sequences.

15. Method for producing a polypeptide according to one of Claims 1 to 3, comprising the culture of a cell which contains a nucleic acid according to one of Claims 4 to 7 or a vector according to Claim 8 or 9, under conditions for expressing said nucleic acid, and the recovery of the peptide compound produced.

16. Cell containing a nucleic acid according to one of Claims 4 to 7 or a vector according to Claim 8 or 9.

17. Nonhuman mammal comprising in its cells a nucleic acid according to one of Claims 4 to 7.

18. Antibodies or antibody fragments or derivatives that are directed against a polypeptide according to one of Claims 1 to 3.

## Patentansprüche

1. Polypeptid, das mit Parkin interagiert und das über seine Gesamtlänge eine Identität von mindestens 95% mit der Sequenz SEQ ID NO: 2 aufweist.

2. Polypeptid, das eine der Sequenzen SEQ ID NO: 13 und SEQ ID NO: 43 aufweist.

3. Polypeptid, das eine der Sequenzen SEQ ID NO: 15 und SEQ ID NO: 45 aufweist.

4. Nukleinsäure, die für ein Polypeptid nach einem der Ansprüche 1 bis 3 kodiert.

5. Nukleinsäure nach Anspruch 4, **dadurch gekennzeichnet, dass** sie eine Identität von mindestens 95% zu der Sequenz SEQ ID NO: 1 aufweist.

6. Nukleinsäure nach Anspruch 4, **dadurch gekennzeichnet, dass** sie eine der Sequenzen SEQ ID NO: 12 und SEQ ID NO: 42 aufweist.

7. Nukleinsäure nach Anspruch 4, **dadurch gekennzeichnet, dass** sie eine der Sequenzen SEQ ID NO: 14 und SEQ ID NO: 44 aufweist.

8. Vektor, umfassend eine Nukleinsäure nach einem der Ansprüche 4 bis 7.

9. Defizientes rekombinantes Virus, umfassend eine Nukleinsäure nach einem der Ansprüche 4 bis 7.

10. Pharmazeutische Zusammensetzung, umfassend mindestens ein Polypeptid nach einem der Ansprüche 1 bis 3.

11. Pharmazeutische Zusammensetzung, umfassend mindestens eine Nukleinsäure nach einem der Ansprüche 4 bis 7 oder einen Vektor nach einem der Ansprüche 8 oder 9.

12. Zusammensetzung nach einem der Ansprüche 10 und 11 für die Behandlung von neurodegenerativen Erkrankungen.

13. Verfahren für das Screening oder die Charakterisierung von Molekülen für die Behandlung von neurodegenerativen Erkrankungen, umfassend einen Selektionsschritt für Moleküle, die fähig sind, an die Sequenz SEQ ID NO: 2 oder ein Fragment davon zu binden.

14. Verfahren für das Screening oder die Charakterisierung von Molekülen für die Behandlung von neurodegenerativen Erkrankungen, umfassend einen Selektionsschritt für Moleküle, die fähig sind, an eine Sequenz ausgewählt aus den Sequenzen SEQ ID NO: 13, SEQ ID NO: 15, SEQ ID NO: 43 und SEQ ID NO: 45 oder ein Fragment davon zu binden.

15. Verfahren zur Herstellung eines Polypeptids nach einem der Ansprüche 1 bis 3, bei dem man eine Zelle enthaltend eine Nukleinsäure nach einem der Ansprüche 4 bis 7 oder einen Vektor nach Anspruch 8 oder 9 unter Expressionsbedingungen für diese Nukleinsäure kultiviert und die produzierte Peptidverbindung gewinnt.

16. Zelle, enthaltend eine Nukleinsäure nach einem der Ansprüche 4 bis 7 oder einen Vektor nach Anspruch 8 oder 9.

17. Nichtmenschliches Säugetier, umfassend in seinen Zellen eine Nukleinsäure nach einem der Ansprüche 4 bis 7.

18. Antikörper, Fragmente oder Derivate von Antikörpern, die gegen ein Polypeptid nach einem der Ansprüche 1 bis 3 gerichtet sind.


### LISTE DE SEQUENCES

<110> AVENTIS PHARMA
   INSTITUT NATIONAL DE LA SANTE ET DE LA RECHERCHE M
<120> COMPOSES CAPABLES DE MODULER L'ACTIVITE DE LA PARKINE, SEQUENCES NUCLEOTIDIQUES ET UTILISATIONS
<130> PRJ00004
<140>
   <141>
<160> 46
<170> PatentIn Ver. 2.1
<210> 1
   <211> 1313
   <212> ADN
   <213> Homo sapiens
<220>
   <221> CDS
   <222> (1)..(1032)
<400> 1
   cag aat ctc cca tcc agt ccg gca ccc agt acc ata ttc tct gga ggt    48
   Gln Asn Leu Pro Ser Ser Pro Ala Pro Ser Thr Ile Phe Ser Gly Gly
     1               5                  10                  15
   ttt aga cac gga agt tta att agc att gac agc acc tgt aca gag atg    96
   Phe Arg His Gly Ser Leu Ile Ser Ile Asp Ser Thr Cys Thr Glu Met
                20                  25                  30
   ggc aat ttt gac aat gct aat gtc act gga gaa ata gaa ttt gcc att   144
   Gly Asn Phe Asp Asn Ala Asn Val Thr Gly Glu Ile Glu Phe Ala Ile
            35                  40                  45
   cat tat tgc ttc aaa acc cat tct tta gaa ata tgc atc aag gcc tgt   192
   His Tyr Cys Phe Lys Thr His Ser Leu Glu Ile Cys Ile Lys Ala Cys
        50                  55                  60
   aag aac ctt gcc tat gga gaa gaa aag aag aaa aag tgc aat ccg tat   240
   Lys Asn Leu Ala Tyr Gly Glu Glu Lys Lys Lys Lys Cys Asn Pro Tyr
    65                  70                  75                  80
   gtg aag acc tac ctg ttg ccc gac aga tcc tcc cag gga aag cgc aag   288
   Val Lys Thr Tyr Leu Leu Pro Asp Arg Ser Ser Gln Gly Lys Arg Lys
   85 90 95
   act gga gtc caa agg aac acc gtg gac ccg acc ttt cag gag acc ttg 336
   Thr Gly Val Gln Arg Asn Thr Val Asp Pro Thr Phe Gln Glu Thr Leu
   100 105 110
   aag tat cag gtg gcc cct gcc cag ctg gtg acc cgg cag ctg cag gtc 384
   Lys Tyr Gln Val Ala Pro Ala Gln Leu Val Thr Arg Gln Leu Gln Val
   115 120 125
   tcg gtg tgg cat ctg ggc acg ctg gcc cgg aga gtg ttt ctt gga gaa 432
   Ser Val Trp His Leu Gly Thr Leu Ala Arg Arg Val Phe Leu Gly Glu
   130 135 140
   gtg atc att tct ctg gcc acg tgg gac ttt gaa gac agc aca aca cag 480
   Val Ile Ile Ser Leu Ala Thr Trp Asp Phe Glu Asp Ser Thr Thr Gln
   145 150 155 160
   tcc ttc cgc tgg cat ccg ctc cgg gcc aag gcg gag aaa tac gaa gac 528
   Ser Phe Arg Trp His Pro Leu Arg Ala Lys Ala Glu Lys Tyr Glu Asp
   165 170 175
   agc gtt cct cag agt aat gga gag ctc aca gtc cgg gct aag ctg gtt 576
   Ser Val Pro Gln Ser Asn Gly Glu Leu Thr Val Arg Ala Lys Leu Val
   180 185 190
   ctc cct tca cgg ccc aga aaa ctc caa gag gct caa gaa ggg aca gat 624
   Leu Pro Ser Arg Pro Arg Lys Leu Gln Glu Ala Gln Glu Gly Thr Asp
   195 200 205
   cag cca tca ctt cat ggt caa ctt tgt ttg gta gtg cta gga gcc aag 672
   Gln Pro Ser Leu His Gly Gln Leu Cys Leu Val Val Leu Gly Ala Lys
   210 215 220
   aat tta cct gtg cgg cca gat ggc acc ttg aac tca ttt gtt aag ggc 720
   Asn Leu Pro Val Arg Pro Asp Gly Thr Leu Asn Ser Phe Val Lys Gly
   225 230 235 240
   tgt ctc act ctg cca gac caa caa aaa ctg aga ctg aag tcg cca gtc 768
   Cys Leu Thr Leu Pro Asp Gln Gln Lys Leu Arg Leu Lys Ser Pro Val
   245 250 255
   ctg agg aag cag gct tgc ccc cag tgg aaa cac tca ttt gtc ttc agt 816
   Leu Arg Lys Gln Ala Cys Pro Gln Trp Lys His Ser Phe Val Phe Ser
   260 265 270
   ggc gta acc cca gct cag ctg agg cag tcg agc ttg gag tta act gtc 864
   Gly Val Thr Pro Ala Gln Leu Arg Gln Ser Ser Leu Glu Leu Thr Val
           275                 280                 285
   tgg gat cag gcc ctc ttt gga atg aat gac cgc ttg ctt gga gga acc    912
   Trp Asp Gln Ala Leu Phe Gly Met Asn Asp Arg Leu Leu Gly Gly Thr
       290                 295                 300
   aga ctt ggt tca aag gga gac aca gct gtt ggc ggg gat gca tgc tca    960
   Arg Leu Gly Ser Lys Gly Asp Thr Ala Val Gly Gly Asp Ala Cys Ser
   305                 310                 315                 320
   cta tcg aag ctc cag tgg cag aaa gtc ctt tcc agc ccc aat cta tgg   1008
   Leu Ser Lys Leu Gln Trp Gln Lys Val Leu Ser Ser Pro Asn Leu Trp
                   325                 330                 335
   aca gac atg act ctt gtc ctg cac tgacatgaag gcctcaaggt tccaggttgc  1062
   Thr Asp Met Thr Leu Val Leu His
               340
   agcaggcgtg aggcactgtg cgtctgcaga ggggctacga accaggtgca gggtcccagc 1122
   tggagacccc tttgaccttg agcagtctcc atctgcggcc ctgtcccatg gcttaaccgc 1182
   ctattggtat ctgtgtatat ttacgttaaa cacaattatg ttacctaagc ctctggtggg 1242
   ttatctcctc tttgagatgt agaaaatggc cagattttaa taaacgttgt tacccatgaa 1302
   aaaaaaaaaa a                                                      1313
<210> 2
   <211> 344
   <212> PRT
   <213> Homo sapiens
<400> 2
   Gln Asn Leu Pro Ser Ser Pro Ala Pro Ser Thr Ile Phe Ser Gly Gly
    1               5                  10                  15
   Phe Arg His Gly Ser Leu Ile Ser Ile Asp Ser Thr Cys Thr Glu Met
               20                  25                  30
   Gly Asn Phe Asp Asn Ala Asn Val Thr Gly Glu Ile Glu Phe Ala Ile
           35                  40                  45
   His Tyr Cys Phe Lys Thr His Ser Leu Glu Ile Cys Ile Lys Ala Cys
       50                  55                  60
   Lys Asn Leu Ala Tyr Gly Glu Glu Lys Lys Lys Lys Cys Asn Pro Tyr
   65 70 75 80
   Val Lys Thr Tyr Leu Leu Pro Asp Arg Ser Ser Gln Gly Lys Arg Lys
   85 90 95
   Thr Gly Val Gln Arg Asn Thr Val Asp Pro Thr Phe Gln Glu Thr Leu
   100 105 110
   Lys Tyr Gln Val Ala Pro Ala Gln Leu Val Thr Arg Gln Leu Gln Val
   115 120 125
   Ser Val Trp His Leu Gly Thr Leu Ala Arg Arg Val Phe Leu Gly Glu
   130 135 140
   Val Ile Ile Ser Leu Ala Thr Trp Asp Phe Glu Asp Ser Thr Thr Gln
   145 150 155 160
   Ser Phe Arg Trp His Pro Leu Arg Ala Lys Ala Glu Lys Tyr Glu Asp
   165 170 175
   Ser Val Pro Gln Ser Asn Gly Glu Leu Thr Val Arg Ala Lys Leu Val
   180 185 190
   Leu Pro Ser Arg Pro Arg Lys Leu Gln Glu Ala Gln Glu Gly Thr Asp
   195 200 205
   Gln Pro Ser Leu His Gly Gln Leu Cys Leu Val Val Leu Gly Ala Lys
   210 215 220
   Asn Leu Pro Val Arg Pro Asp Gly Thr Leu Asn Ser Phe Val Lys Gly
   225 230 235 240
   Cys Leu Thr Leu Pro Asp Gln Gln Lys Leu Arg Leu Lys Ser Pro Val
   245 250 255
   Leu Arg Lys Gln Ala Cys Pro Gln Trp Lys His Ser Phe Val Phe Ser
   260 265 270
   Gly Val Thr Pro Ala Gln Leu Arg Gln Ser Ser Leu Glu Leu Thr Val
   275 280 285
   Trp Asp Gln Ala Leu Phe Gly Met Asn Asp Arg Leu Leu Gly Gly Thr
   290 295 300
   Arg Leu Gly Ser Lys Gly Asp Thr Ala Val Gly Gly Asp Ala Cys Ser
   305 310 315 320
   Leu Ser Lys Leu Gln Trp Gln Lys Val Leu Ser Ser Pro Asn Leu Trp
                   325                     330                 335
   Thr Asp Met Thr Leu Val Leu His
               340
<210> 3
   <211> 471
   <212> ADN
   <213> Homo sapiens
<220>
   <221> CDS
   <222> (1)..(471)
<400> 3
   gga agt cca gca ggt aga tca atc tac aac agc ttt tat gtg tat tgc    48
   Gly Ser Pro Ala Gly Arg Ser Ile Tyr Asn Ser Phe Tyr Val Tyr Cys
     1               5                  10                  15
   aaa ggc ccc tgt caa aga gtg cag ccg gga aaa ctc agg gta cag tgc    96
   Lys Gly Pro Cys Gln Arg Val Gln Pro Gly Lys Leu Arg Val Gln Cys
                20                  25                  30
   agc acc tgc agg cag gca acg ctc acc ttg acc cag ggt cca tct tgc   144
   Ser Thr Cys Arg Gln Ala Thr Leu Thr Leu Thr Gln Gly Pro Ser Cys
            35                  40                  45
   tgg gat gat gtt tta att cca aac cgg atg agt ggt gaa tgc caa tcc   192
   Trp Asp Asp Val Leu Ile Pro Asn Arg Met Ser Gly Glu Cys Gln Ser
        50                  55                  60
   cca cac tgc cct ggg act agt gca gaa ttt ttc ttt aaa tgt gga gca   240
   Pro His Cys Pro Gly Thr Ser Ala Glu Phe Phe Phe Lys Cys Gly Ala
    65                  70                  75                  80
   cac ccc acc tct gac aag gaa aca tca gta gct ttg cac ctg atc gca   288
   His Pro Thr Ser Asp Lys Glu Thr Ser Val Ala Leu His Leu Ile Ala
                    85                  90                  95
   aca aat agt cgg aac atc act tgc att acg tgc aca gac gtc agg agc   336
   Thr Asn Ser Arg Asn Ile Thr Cys Ile Thr Cys Thr Asp Val Arg Ser
               100                 105                 110
   ccc gtc ctg gtt ttc cag tgc aac tcc cgc cac gtg att tgc tta gac   384
   Pro Val Leu Val Phe Gln Cys Asn Ser Arg His Val Ile Cys Leu Asp
           115                 120                 125
   tgt ttc cac tta tac tgt gtg aca aga ctc aat gat cgg cag ttt gtt   432
   Cys Phe His Leu Tyr Cys Val Thr Arg Leu Asn Asp Arg Gln Phe Val
       130                 135                 140
   cac gac cct caa ctt ggc tac tcc ctg cct tgt gtg tag               471
   His Asp Pro Gln Leu Gly Tyr Ser Leu Pro Cys Val
   145                 150                 155
<210> 4
   <211> 156
   <212> PRT
   <213> Homo sapiens
<400> 4
   Gly Ser Pro Ala Gly Arg Ser Ile Tyr Asn Ser Phe Tyr Val Tyr Cys
    1               5                  10                  15
   Lys Gly Pro Cys Gln Arg Val Gln Pro Gly Lys Leu Arg Val Gln Cys
               20                  25                  30
   Ser Thr Cys Arg Gln Ala Thr Leu Thr Leu Thr Gln Gly Pro Ser Cys
           35                  40                  45
   Trp Asp Asp Val Leu Ile Pro Asn Arg Met Ser Gly Glu Cys Gln Ser
       50                  55                  60
   Pro His Cys Pro Gly Thr Ser Ala Glu Phe Phe Phe Lys Cys Gly Ala
   65                  70                  75                  80
   His Pro Thr Ser Asp Lys Glu Thr Ser Val Ala Leu His Leu Ile Ala
                   85                  90                  95
   Thr Asn Ser Arg Asn Ile Thr Cys Ile Thr Cys Thr Asp Val Arg Ser
               100                 105                 110
   Pro Val Leu Val Phe Gln Cys Asn Ser Arg His Val Ile Cys Leu Asp
           115                 120                 125
   Cys Phe His Leu Tyr Cys Val Thr Arg Leu Asn Asp Arg Gln Phe Val
       130                 135                 140
   His Asp Pro Gln Leu Gly Tyr Ser Leu Pro Cys Val
   145                 150                 155
<210> 5
   <211> 27
   <212> ADN
   <213> Séquence artificielle
<220>
   <223> Description de la séquence artificielle:Oligonucleotide
<400> 5
   ttaagaattc ggaagtccag caggtag 27
<210> 6
   <211> 29
   <212> ADN
   <213> Séquence artificielle
<220>
   <223> Description de la séquence artificielle:Oligonucleotide
<400> 6
   attaggatcc ctacacacaa ggcagggag 29
<210> 7
   <211> 19
   <212> ADN
   <213> Séquence artificielle
<220>
   <223> Description de la séquence
   artificielle:Oligonucleotide
<400> 7
   gcgtttggaa tcactacag 19
<210> 8
   <211> 17
   <212> ADN
   <213> Séquence artificielle
<220>
   <223> Description de la séquence
   artificielle:Oligonucleotide
<400> 8
   ggtctcggtg tggcatc 17
<210> 9
   <211> 18
   <212> ADN
   <213> Séquence artificielle
<220>
   <223> Description de la séquence
   artificielle:Oligonucleotide
<400> 9
   ccgcttgctt ggaggaac 18
<210> 10
   <211> 18
   <212> ADN
   <213> Séquence artificielle
<220>
   <223> Description de la séquence
   artificielle:Oligonucleotide
<400> 10
   cgtatttctc cgccttgg 18
<210> 11
   <211> 28
   <212> ADN
   <213> Séquence artificielle
<220>
   <223> Description de la séquence
   artificielle:Oligonucleotide
<400> 11
   aatagctcga gtcagtgcag gacaagag 28
<210> 12
   <211> 2347
   <212> ADN
   <213> Homo sapiens
<400> 12
   ggccttgggg cactgaggga tgccagttct gcctgttcat ctggaacctg gatctaagga 60
   gggaagaggc gttgcccctg ctggcatagt caggtaccag cccagccagg tattgaacgg 120
   gctgagcttt tcatgatggt tcctgctgac ctggaaacat cttaaatgga agggcgtgag 180
   cgcttggtcc atgcagtgaa gctcttccaa cctgggtcaa cgaaaacgga gaagaaatgg 240
   cccaagaaat agatctgagt gctctcaagg agttagaacg cgaggccatt ctccaggtcc 300
   tgtaccgaga ccaggcggtt caaaacacag aggaggagag gacacggaaa ctgaaaacac 360
   acctgcagca tctccggtgg aaaggagcga agaacacgga ctgggagcac aaagagaagt 420
   gctgtgcgcg ctgccagcag gtgctggggt tcctgctgca ccggggcgcc gtgtgccggg 480
   gctgcagcca ccgcgtgtgt gcccagtgcc gagtgttcct gagggggacc catgcctgga 540
   agtgcacggt gtgcttcgag gacaggaatg tcaaaataaa aactggagaa tggttctatg 600
   aggaacgagc caagaaattt ccaactggag gcaaacatga gacagttgga gggcagctct 660
   tgcaatctta tcagaagctg agcaaaattt ctgtggttcc tcctactcca cctcctgtca 720
   gcgagagcca gtgcagccgc agtcctggca ggttacagga atttggtcag tttagaggat 780
   ttaataagtc cgtggaaaat ttgtttctgt ctcttgctac ccacgtgaaa aagctctcca 840
   aatcccagaa tgatatgact tctgagaagc atcttctcgc cacgggcccc aggcagtgtg 900
   tgggacagac agagagacgg agccagtctg acactgcggt caacgtcacc accaggaagg 960
   tcagtgcacc agatattctg aaacctctca atcaagagga tcccaaatgc tctactaacc 1020
   ctattttgaa gcaacagaat ctcccatcca gtccggcacc cagtaccata ttctctggag 1080
   gttttagaca cggaagttta attagcattg acagcacctg tacagagatg ggcaattttg 1140
   acaatgctaa tgtcactgga gaaatagaat ttgccattca ttattgcttc aaaacccatt 1200
   ctttagaaat atgcatcaag gcctgtaaga accttgccta tggagaagaa aagaagaaaa 1260
   agtgcaatcc gtatgtgaag acctacctgt tgcccgacag atcctcccag ggaaagcgca 1320
   agactggagt ccaaaggaac accgtggacc cgacctttca ggagaccttg aagtatcagg 1380
   tggcccctgc ccagctggtg acccggcagc tgcaggtctc ggtgtggcat ctgggcacgc 1440
   tggcccggag agtgtttctt ggagaagtga tcattcctct ggccacgtgg gactttgaag 1500
   acagcacaac acagtccttc cgctggcatc cgctccgggc caaggcggag aaatacgaag 1560
   acagcgttcc tcagagtaat ggagagctca cagtccgggc taagctggtt ctcccttcac 1620
   ggcccagaaa actccaagag gctcaagaag ggacagatca gccatcactt catggtcaac 1680
   tttgtttggt agtgctagga gccaagaatt tacctgtgcg gccagatggc accttgaact 1740
   catttgttaa gggctgtctc actctgccag accaacaaaa actgagactg aagtcgccag 1800
   tcctgaggaa gcaggcttgc ccccagtgga aacactcatt tgtcttcagt ggcgtaaccc 1860
   cagctcagct gaggcagtcg agcttggagt taactgtctg ggatcaggcc ctctttggaa 1920
   tgaacgaccg cttgcttgga ggaaccagac ttggttcaaa gggagacaca gctgttggcg 1980
   gggatgcatg ctcacaatcg aagctccagt ggcagaaagt cctttccagc cccaatctat 2040
   ggacagacat gactcttgtc ctgcactgac atgaaggcct caaggttcca ggttgcagca 2100
   ggcgtgaggc actgtgcgtc tgcagagggg ctacgaacca ggtgcagggt cccagctgga 2160
   gacccctttg accttgagca gtctccatct gcggccctgt cccatggctt aaccgcctat 2220
   tggtatctgt gtatatttac gttaaacaca attatgttac ctaagcctct ggtgggttat 2280
   ctcctctttg agatgtagaa aatggccaga ttttaataaa cgttgttacc catgaaaaaa 2340
   aaaaaaa                                                           2347
<210> 13
   <211> 610
   <212> PRT
   <213> Homo sapiens
<400> 13
   Met Ala Gln Glu Ile Asp Leu Ser Ala Leu Lys Glu Leu Glu Arg Glu
    1               5                  10                  15
   Ala Ile Leu Gln Val Leu Tyr Arg Asp Gln Ala Val Gln Asn Thr Glu
               20                  25                  30
   Glu Glu Arg Thr Arg Lys Leu Lys Thr His Leu Gln His Leu Arg Trp
   35 40 45
   Lys Gly Ala Lys Asn Thr Asp Trp Glu His Lys Glu Lys Cys Cys Ala
   50 55 60
   Arg Cys Gln Gln Val Leu Gly Phe Leu Leu His Arg Gly Ala Val Cys
   65 70 75 80
   Arg Gly Cys Ser His Arg Val Cys Ala Gln Cys Arg Val Phe Leu Arg
   85 90 95
   Gly Thr His Ala Trp Lys Cys Thr Val Cys Phe Glu Asp Arg Asn Val
   100 105 110
   Lys Ile Lys Thr Gly Glu Trp Phe Tyr Glu Glu Arg Ala Lys Lys Phe
   115 120 125
   Pro Thr Gly Gly Lys His Glu Thr Val Gly Gly Gln Leu Leu Gln Ser
   130 135 140
   Tyr Gln Lys Leu Ser Lys Ile Ser Val Val Pro Pro Thr Pro Pro Pro
   145 150 155 160
   Val Ser Glu Ser Gln Cys Ser Arg Ser Pro Gly Arg Leu Gln Glu Phe
   165 170 175
   Gly Gln Phe Arg Gly Phe Asn Lys Ser Val Glu Asn Leu Phe Leu Ser
   180 185 190
   Leu Ala Thr His Val Lys Lys Leu Ser Lys Ser Gln Asn Asp Met Thr
   195 200 205
   Ser Glu Lys His Leu Leu Ala Thr Gly Pro Arg Gln Cys Val Gly Gln
   210 215 220
   Thr Glu Arg Arg Ser Gln Ser Asp Thr Ala Val Asn Val Thr Thr Arg
   225 230 235 240
   Lys Val Ser Ala Pro Asp Ile Leu Lys Pro Leu Asn Gln Glu Asp Pro
   245 250 255
   Lys Cys Ser Thr Asn Pro Ile Leu Lys Gln Gln Asn Leu Pro Ser Ser
   260 265 270
   Pro Ala Pro Ser Thr Ile Phe Ser Gly Gly Phe Arg His Gly Ser Leu
   275 280 285
   Ile Ser Ile Asp Ser Thr Cys Thr Glu Met Gly Asn Phe Asp Asn Ala
   290 295 300
   Asn Val Thr Gly Glu Ile Glu Phe Ala Ile His Tyr Cys Phe Lys Thr
   305 310 315 320
   His Ser Leu Glu Ile Cys Ile Lys Ala Cys Lys Asn Leu Ala Tyr Gly
   325 330 335
   Glu Glu Lys Lys Lys Lys Cys Asn Pro Tyr Val Lys Thr Tyr Leu Leu
   340 345 350
   Pro Asp Arg Ser Ser Gln Gly Lys Arg Lys Thr Gly Val Gln Arg Asn
   355 360 365
   Thr Val Asp Pro Thr Phe Gln Glu Thr Leu Lys Tyr Gln Val Ala Pro
   370 375 380
   Ala Gln Leu Val Thr Arg Gln Leu Gln Val Ser Val Trp His Leu Gly
   385 390 395 400
   Thr Leu Ala Arg Arg Val Phe Leu Gly Glu Val Ile Ile Pro Leu Ala
   405 410 415
   Thr Trp Asp Phe Glu Asp Ser Thr Thr Gln Ser Phe Arg Trp His Pro
   420 425 430
   Leu Arg Ala Lys Ala Glu Lys Tyr Glu Asp Ser Val Pro Gln Ser Asn
   435 440 445
   Gly Glu Leu Thr Val Arg Ala Lys Leu Val Leu Pro Ser Arg Pro Arg
   450 455 460
   Lys Leu Gln Glu Ala Gln Glu Gly Thr Asp Gln Pro Ser Leu His Gly
   465 470 475 480
   Gln Leu Cys Leu Val Val Leu Gly Ala Lys Asn Leu Pro Val Arg Pro
   485 490 495
   Asp Gly Thr Leu Asn Ser Phe Val Lys Gly Cys Leu Thr Leu Pro Asp
   500 505 510
   Gln Gln Lys Leu Arg Leu Lys Ser Pro Val Leu Arg Lys Gln Ala Cys
   515 520 525
   Pro Gln Trp Lys His Ser Phe Val Phe Ser Gly Val Thr Pro Ala Gln
   530 535 540
   Leu Arg Gln Ser Ser Leu Glu Leu Thr Val Trp Asp Gln Ala Leu Phe
   545 550 555 560
   Gly Met Asn Asp Arg Leu Leu Gly Gly Thr Arg Leu Gly Ser Lys Gly
   565 570 575
   Asp Thr Ala Val Gly Gly Asp Ala Cys Ser Gln Ser Lys Leu Gln Trp
   580 585 590
   Gln Lys Val Leu Ser Ser Pro Asn Leu Trp Thr Asp Met Thr Leu Val
   595 600 605
   Leu His
   610
<210> 14
   <211> 1648
   <212> ADN
   <213> Homo sapiens
<400> 14
   gaaatcatgc ccctcgtaga gcagcaggtc caagcagggc tgctggctat ttttccaaaa 60
   agtgaggcag tttaaaaaaa aggcggagaa ctagaattat agaataatgg cacattttgt 120
   gtatttgtaa aactaacggc ttgcatggtt cacaacccat ttcttatgcc tgtgttttcc 180
   ttggcagcaa aatttctgtg gttcctccta ctccacctcc tgtcagcgag agccagtgca 240
   gccgcagtcc tggcaggaag gtcagtgcac cagatattct gaaacctctc aatcaagagg 300
   atcccaaatg ctctactaac cctattttga agcaacagaa tctcccatcc agtccggcac 360
   ccagtaccat attctctgga ggttttagac acggaagttt aattagcatt gacagcacct 420
   gtacagagat gggcaatttt gacaatgcta atgtcactgg agaaatagaa tttgccattc 480
   attattgctt caaaacccat tctttagaaa tatgcatcaa ggcctgtaag aaccttgcct 540
   atggagaaga aaagaagaaa aagtgcaatc cgtatgtgaa gacctacctg ttgcccgaca 600
   gatcctccca gggaaagcgc aagactggag tccaaaggaa caccgtggac ccgacctttc 660
   aggagacctt gaagtatcag gtggcccctg cccagctggt gacccggcag ctgcaggtct 720
   cggtgtggca tctgggcacg ctggcccgga gagtgtttct tggagaagtg atcattcctc 780
   tggccacgtg ggactttgaa gacagcacaa cacagtcctt ccgctggcat ccgctccggg 840
   ccaaggcgga gaaatacgaa gacagcgttc ctcagagtaa tggagagctc acagtccggg 900
   ctaagctggt tctcccttca cggcccagaa aactccaaga ggctcaagaa gggacagatc 960
   agccatcact tcatggtcaa ctttgtttgg tagtgctagg agccaagaat ttacctgtgc 1020
   ggccagatgg caccttgaac tcatttgtta agggctgtct cactctgcca gaccaacaaa 1080
   aactgagact gaagtcgcca gtcctgagga agcaggcttg cccccagtgg aaacactcat 1140
   ttgtcttcag tggcgtaacc ccagctcagc tgaggcagtc gagcttggag ttaactgtct 1200
   gggatcaggc cctctttgga atgaacgacc gcttgcttgg aggaaccaga cttggttcaa 1260
   agggagacac agctgttggc ggggatgcat gctcacaatc gaagctccag tggcagaaag 1320
   tcctttccag ccccaatcta tggacagaca tgactcttgt cctgcactga catgaaggcc 1380
   tcaaggttcc aggttgcagc aggcgtgagg cactgtgcgt ctgcagaggg gctacgaacc 1440
   aggtgcaggg tcccagctgg agaccccttt gaccttgagc agtctccatc tgcggccctg 1500
   tcccatggct taaccgccta ttggtatctg tgtatattta cgttaaacac aattatgtta 1560
   cctaagcctc tggtgggtta tctcctcttt gagatgtaga aaatggccag attttaataa 1620
   acgttgttac ccatgaaaaa aaaaaaaa 1648
<210> 15
   <211> 313
   <212> PRT
   <213> Homo sapiens
<400> 15
<210> 16
   <211> 19
   <212> ADN
   <213> Séquence artificielle
<220>
   <223> Description de la séquence artificielle:
   oligonucleotide
<400> 16
   ccagttctgc ctgttcatc 19
<210> 17
   <211> 20
   <212> ADN
   <213> Séquence artificielle
<220>
   <223> Description de la séquence artificielle:
   oligonucleotide
<400> 17
   ttcaaaacac agaggaggag 20
<210> 18
   <211> 20
   <212> ADN
   <213> Séquence artificielle
<220>
   <223> Description de la séquence artificielle:
   oligonucleotide
<400> 18
   gaatttggtc agtttagagg 20
<210> 19
   <211> 26
   <212> ADN
   <213> Séquence artificielle
<220>
   <223> Description de la séquence artificielle:
   oligonucleotide
<400> 19
   ttctgggatt tggagagctt tttcac 26
<210> 20
   <211> 22
   <212> ADN
   <213> Séquence artificielle
<220>
   <223> Description de la séquence artificielle:
   oligonucleotide
<400> 20
   tctgtctgtc ccacacactg cc 22
<210> 21
   <211> 19
   <212> ADN
   <213> Séquence artificielle
<220>
   <223> Description de la séquence artificielle:
   oligonucleotide
<400> 21
   gactggctcc gtctctctg 19
<210> 22
   <211> 21
   <212> ADN
   <213> Séquence artificielle
<220>
   <223> Description de la séquence artificielle:
   oligonucleotide
<400> 22
   aagcaacaga atctcccatc c 21
<210> 23
   <211> 21
   <212> ADN
   <213> Séquence artificielle
<220>
   <223> Description de la séquence artificielle:
   oligonucleotide
<400> 23
   gcattgtcaa aattgcccat c 21
<210> 24
   <211> 20
   <212> ADN
   <213> Séquence artificielle
<220>
   <223> Description de la séquence artificielle:
   oligonucleotide
<400> 24
   aggcggagaa atacgaagac 20
<210> 25
   <211> 22
   <212> ADN
   <213> Séquence artificielle
<220>
   <223> Description de la séquence artificielle:
   oligonucleotide
<400> 25
   gcagagtgag acagccctta ac 22
<210> 26
   <211> 24
   <212> ADN
   <213> Séquence artificielle
<220>
   <223> Description de la séquence artificielle:
   oligonucleotide
<400> 26
   cttcctcagg actggcgact tcag 24
<210> 27
   <211> 24
   <212> ADN
   <213> Séquence artificielle
<220>
   <223> Description de la séquence artificielle:
   oligonucleotide
<400> 27
   caagcggtcg ttcattccaa agag 24
<210> 28
   <211> 22
   <212> ADN
   <213> Séquence artificielle
<220>
   <223> Description de la séquence artificielle:
   oligonucleotide
<400> 28
   aagaggagat aacccaccag ag 22
<210> 29
   <211> 20
   <212> ADN
   <213> Séquence artificielle
<220>
   <223> Description de la séquence artificielle:
   oligonucleotide
<400> 29
   agggctgctg gctatttttc 20
<210> 30
   <211> 19
   <212> ADN
   <213> Séquence artificielle
<220>
   <223> Description de la séquence artificielle:
   oligonucleotide
<400> 30
   taagaaatgg gttgtgaac 19
<210> 31
   <211> 21
   <212> ADN
   <213> Séquence artificielle
<220>
   <223> Description de la séquence artificielle:
   oligonucleotide
<400> 31
   aagcaacaga atctcccatc c 21
<210> 32
   <211> 21
   <212> ADN
   <213> Séquence artificielle
<220>
   <223> Description de la séquence artificielle:
   oligonucleotide
<400> 32
   gcattgtcaa aattgcccat c 21
<210> 33
   <211> 20
   <212> ADN
   <213> Séquence artificielle
<220>
   <223> Description de la séquence artificielle:
   oligonucleotide
<400> 33
   aggcggagaa atacgaagac 20
<210> 34
   <211> 22
   <212> ADN
   <213> Séquence artificielle
<220>
   <223> Description de la séquence artificielle:
   oligonucleotide
<400> 34
   gcagagtgag acagccctta ac 22
<210> 35
   <211> 24
   <212> ADN
   <213> Séquence artificielle
<220>
   <223> Description de la séquence artificielle:
   oligonucleotide
<400> 35
   cttcctcagg actggcgact tcag 24
<210> 36
   <211> 24
   <212> ADN
   <213> Séquence artificielle
<220>
   <223> Description de la séquence artificielle:
   oligonucleotide
<400> 36
   caagcggtcg ttcattccaa agag 24
<210> 37
   <211> 22
   <212> ADN
   <213> Séquence artificielle
<220>
   <223> Description de la séquence artificielle:
   oligonucleotide
<400> 37
   aagaggagat aacccaccag ag 22
<210> 38
   <211> 18
   <212> ADN
   <213> Séquence artificielle
<220>
   <223> Description de la séquence artificielle:
   oligonucleotide
<400> 38
   aatggaaggg cgtgacgc 18
<210> 39
   <211> 21
   <212> ADN
   <213> Séquence artificielle
<220>
   <223> Description de la séquence artificielle:
   oligonucleotide
<400> 39
   cctcacgcct gctgcaacct g 21
<210> 40
   <211> 31
   <212> ADN
   <213> Séquence artificielle
<220>
   <223> Description de la séquence artificielle:
   oligonucleotide
<400> 40
   gcacgaattc atggcccaag aaatagatct g 31
<210> 41
   <211> 24
   <212> ADN
   <213> Séquence artificielle
<220>
   <223> Description de la séquence artificielle:
   oligonucleotide
<400> 41
   ctgtcttcgt atttctccgc cttg 24
<210> 42
   <211> 2347
   <212> ADN
   <213> Homo sapiens
<400> 42
   ggccttgggg cactgaggga tgccagttct gcctgttcat ctggaacctg gatctaagga 60
   gggaagaggc gttgcccctg ctggcatagt caggtaccag cccagccagg tattgaacgg 120
   gctgagcttt tcatgatggt tcctgctgac ctggaaacat cttaaatgga agggcgtgag 180
   cgcttggtcc atgcagtgaa gctcttccaa cctgggtcaa cgaaaacgga gaagaaatgg 240
   cccaagaaat agatctgagt gctctcaagg agttagaacg cgaggccatt ctccaggtcc 300
   tgtaccgaga ccaggcggtt caaaacacag aggaggagag gacacggaaa ctgaaaacac 360
   acctgcagca tctccggtgg aaaggagcga agaacacgga ctgggagcac aaagagaagt 420
   gctgtgcgcg ctgccagcag gtgctggggt tcctgctgca ccggggcgcc gtgtgccggg 480
   gctgcagcca ccgcgtgtgt gcccagtgcc gagtgttcct gagggggacc catgcctgga 540
   agtgcacggt gtgcttcgag gacaggaatg tcaaaataaa aactggagaa tggttctatg 600
   aggaacgagc caagaaattt ccaactggag gcaaacatga gacagttgga gggcagctct 660
   tgcaatctta tcagaagctg agcaaaattt ctgtggttcc tcctactcca cctcctgtca 720
   gcgagagcca gtgcagccgc agtcctggca ggttacagga atttggtcag tttagaggat 780
   ttaataagtc cgtggaaaat ttgtttctgt ctcttgctac ccacgtgaaa aagctctcca 840
   aatcccagaa tgatatgact tctgagaagc atcttctcgc cacgggcccc aggcagtgtg 900
   tgggacagac agagagacgg agccagtctg acactgcggt caacgtcacc accaggaagg 960
   tcagtgcacc agatattctg aaacctctca atcaagagga tcccaaatgc tctactaacc 1020
   ctattttgaa gcaacagaat ctcccatcca gtccggcacc cagtaccata ttctctggag 1080
   gttttagaca cggaagttta attagcattg acagcacctg tacagagatg ggcaattttg 1140
   acaatgctaa tgtcactgga gaaatagaat ttgccattca ttattgcttc aaaacccatt 1200
   ctttagaaat atgcatcaag gcctgtaaga accttgccta tggagaagaa aagaagaaaa 1260
   agtgcaatcc gtatgtgaag acctacctgt tgcccgacag atcctcccag ggaaagcgca 1320
   agactggagt ccaaaggaac accgtggacc cgacctttca ggagaccttg aagtatcagg 1380
   tggcccctgc ccagctggtg acccggcagc tgcaggtctc ggtgtggcat ctgggcacgc 1440
   tggcccggag agtgtttctt ggagaagtga tcattcctct ggccacgtgg gactttgaag 1500
   acagcacaac acagtccttc cgctggcatc cgctccgggc caaggcggag aaatacgaag 1560
   acagcgttcc tcagagtaat ggagagctca cagtccgggc taagctggtt ctcccttcac 1620
   ggcccagaaa actccaagag gctcaagaag ggacagatca gccatcactt catggtcaac 1680
   tttgtttggt agtgctagga gccaagaatt tacctgtgcg gccagatggc accttgaact 1740
   catttgttaa gggctgtctc actctgccag accaacaaaa actgagactg aagtcgccag 1800
   tcctgaggaa gcaggcttgc ccccagtgga aacactcatt tgtcttcagt ggcgtaaccc 1860
   cagctcagct gaggcagtcg agcttggagt taactgtctg ggatcaggcc ctctttggaa 1920
   tgaacgaccg cttgcttgga ggaaccagac ttggttcaaa gggagacaca gctgttggcg 1980
   gggatgcatg ctcacaatcg aagctccagt ggcagaaagt cctttccagc cccaatctat 2040
   ggacagacat gactcttgtc ctgcactgac atgaaggcct caaggttcca ggttgcagca 2100
   ggcgtgaggc actgtgcgtc tgcagagggg ctacgaacca ggtgcagggt cccagctgga 2160
   gacccctttg accttgagca gtctccatct gcggccctgt cccatggctt aaccgcctat 2220
   tggtatctgt gtatatttac gttaaacaca attatgttac ctaagcctct ggtgggttat 2280
   ctcctctttg agatgtagaa aatggccaga ttttaataaa cgttgttacc catgaaaaaa 2340
   aaaaaaa                                                           2347
<210> 43
   <211> 610
   <212> PRT
   <213> Homo sapiens
<400> 43
   Met Ala Gln Glu Ile Asp Leu Ser Ala Leu Lys Glu Leu Glu Arg Glu
    1               5                  10                  15
   Ala Ile Leu Gln Val Leu Tyr Arg Asp Gln Ala Val Gln Asn Thr Glu
               20                  25                  30
   Glu Glu Arg Thr Arg Lys Leu Lys Thr His Leu Gln His Leu Arg Trp
           35                  40                  45
   Lys Gly Ala Lys Asn Thr Asp Trp Glu His Lys Glu Lys Cys Cys Ala
       50                  55                  60
   Arg Cys Gln Gln Val Leu Gly Phe Leu Leu His Arg Gly Ala Val Cys
   65                  70                  75                  80
   Arg Gly Cys Ser His Arg Val Cys Ala Gln Cys Arg Val Phe Leu Arg
                   85                  90                  95
   Gly Thr His Ala Trp Lys Cys Thr Val Cys Phe Glu Asp Arg Asn Val
   100 105 110
   Lys Ile Lys Thr Gly Glu Trp Phe Tyr Glu Glu Arg Ala Lys Lys Phe
   115 120 125
   Pro Thr Gly Gly Lys His Glu Thr Val Gly Gly Gln Leu Leu Gln Ser
   130 135 140
   Tyr Gln Lys Leu Ser Lys Ile Ser Val Val Pro Pro Thr Pro Pro Pro
   145 150 155 160
   Val Ser Glu Ser Gln Cys Ser Arg Ser Pro Gly Arg Leu Gln Glu Phe
   165 170 175
   Gly Gln Phe Arg Gly Phe Asn Lys Ser Val Glu Asn Leu Phe Leu Ser
   180 185 190
   Leu Ala Thr His Val Lys Lys Leu Ser Lys Ser Gln Asn Asp Met Thr
   195 200 205
   Ser Glu Lys His Leu Leu Ala Thr Gly Pro Arg Gln Cys Val Gly Gln
   210 215 220
   Thr Glu Arg Arg Ser Gln Ser Asp Thr Ala Val Asn Val Thr Thr Arg
   225 230 235 240
   Lys Val Ser Ala Pro Asp Ile Leu Lys Pro Leu Asn Gln Glu Asp Pro
   245 250 255
   Lys Cys Ser Thr Asn Pro Ile Leu Lys Gln Gln Asn Leu Pro Ser Ser
   260 265 270
   Pro Ala Pro Ser Thr Ile Phe Ser Gly Gly Phe Arg His Gly Ser Leu
   275 280 285
   Ile Ser Ile Asp Ser Thr Cys Thr Glu Met Gly Asn Phe Asp Asn Ala
   290 295 300
   Asn Val Thr Gly Glu Ile Glu Phe Ala Ile His Tyr Cys Phe Lys Thr
   305 310 315 320
   His Ser Leu Glu Ile Cys Ile Lys Ala Cys Lys Asn Leu Ala Tyr Gly
   325 330 335
   Glu Glu Lys Lys Lys Lys Cys Asn Pro Tyr Val Lys Thr Tyr Leu Leu
   340 345 350
   Pro Asp Arg Ser Ser Gln Gly Lys Arg Lys Thr Gly Val Gln Arg Asn
   355 360 365
   Thr Val Asp Pro Thr Phe Gln Glu Thr Leu Lys Tyr Gln Val Ala Pro
   370 375 380
   Ala Gln Leu Val Thr Arg Gln Leu Gln Val Ser Val Trp His Leu Gly
   385 390 395 400
   Thr Leu Ala Arg Arg Val Phe Leu Gly Glu Val Ile Ile Pro Leu Ala
   405 410 415
   Thr Trp Asp Phe Glu Asp Ser Thr Thr Gln Ser Phe Arg Trp His Pro
   420 425 430
   Leu Arg Ala Lys Ala Glu Lys Tyr Glu Asp Ser Val Pro Gln Ser Asn
   435 440 445
   Gly Glu Leu Thr Val Arg Ala Lys Leu Val Leu Pro Ser Arg Pro Arg
   450 455 460
   Lys Leu Gln Glu Ala Gln Glu Gly Thr Asp Gln Pro Ser Leu His Gly
   465 470 475 480
   Gln Leu Cys Leu Val Val Leu Gly Ala Lys Asn Leu Pro Val Arg Pro
   485 490 495
   Asp Gly Thr Leu Asn Ser Phe Val Lys Gly Cys Leu Thr Leu Pro Asp
   500 505 510
   Gln Gln Lys Leu Arg Leu Lys Ser Pro Val Leu Arg Lys Gln Ala Cys
   515 520 525
   Pro Gln Trp Lys His Ser Phe Val Phe Ser Gly Val Thr Pro Ala Gln
   530 535 540
   Leu Arg Gln Ser Ser Leu Glu Leu Thr Val Trp Asp Gln Ala Leu Phe
   545 550 555 560
   Gly Met Asn Asp Arg Leu Leu Gly Gly Thr Arg Leu Gly Ser Lys Gly
   565 570 575
   Asp Thr Ala Val Gly Gly Asp Ala Cys Ser Gln Ser Lys Leu Gln Trp
   580 585 590
   Gln Lys Val Leu Ser Ser Pro Asn Leu Trp Thr Asp Met Thr Leu Val
   595 600 605
   Leu His
   610
<210> 44
   <211> 1648
   <212> ADN
   <213> Homo sapiens
<400> 44
   gaaatcatgc ccctcgtaga gcagcaggtc caagcagggc tgctggctat ttttccaaaa 60
   agtgaggcag tttaaaaaaa aggcggagaa ctagaattat agaataatgg cacattttgt 120
   gtatttgtaa aactaacggc ttgcatggtt cacaacccat ttcttatgcc tgtgttttcc 180
   ttggcagcaa aatttctgtg gttcctccta ctccacctcc tgtcagcgag agccagtgca 240
   gccgcagtcc tggcaggaag gtcagtgcac cagatattct gaaacctctc aatcaagagg 300
   atcccaaatg ctctactaac cctattttga agcaacagaa tctcccatcc agtccggcac 360
   ccagtaccat attctctgga ggttttagac acggaagttt aattagcatt gacagcacct 420
   gtacagagat gggcaatttt gacaatgcta atgtcactgg agaaatagaa tttgccattc 480
   attattgctt caaaacccat tctttagaaa tatgcatcaa ggcctgtaag aaccttgcct 540
   atggagaaga aaagaagaaa aagtgcaatc cgtatgtgaa gacctacctg ttgcccgaca 600
   gatcctccca gggaaagcgc aagactggag tccaaaggaa caccgtggac ccgacctttc 660
   aggagacctt gaagtatcag gtggcccctg cccagctggt gacccggcag ctgcaggtct 720
   cggtgtggca tctgggcacg ctggcccgga gagtgtttct tggagaagtg atcattcctc 780
   tggccacgtg ggactttgaa gacagcacaa cacagtcctt ccgctggcat ccgctccggg 840
   ccaaggcgga gaaatacgaa gacagcgttc ctcagagtaa tggagagctc acagtccggg 900
   ctaagctggt tctcccttca cggcccagaa aactccaaga ggctcaagaa gggacagatc 960
   agccatcact tcatggtcaa ctttgtttgg tagtgctagg agccaagaat ttacctgtgc 1020
   ggccagatgg caccttgaac tcatttgtta agggctgtct cactctgcca gaccaacaaa 1080
   aactgagact gaagtcgcca gtcctgagga agcaggcttg cccccagtgg aaacactcat 1140
   ttgtcttcag tggcgtaacc ccagctcagc tgaggcagtc gagcttggag ttaactgtct 1200
   gggatcaggc cctctttgga atgaacgacc gcttgcttgg aggaaccaga cttggttcaa 1260
   agggagacac agctgttggc ggggatgcat gctcacaatc gaagctccag tggcagaaag 1320
   tcctttccag ccccaatcta tggacagaca tgactcttgt cctgcactga catgaaggcc 1380
   tcaaggttcc aggttgcagc aggcgtgagg cactgtgcgt ctgcagaggg gctacgaacc 1440
   aggtgcaggg tcccagctgg agaccccttt gaccttgagc agtctccatc tgcggccctg 1500
   tcccatggct taaccgccta ttggtatctg tgtatattta cgttaaacac aattatgtta 1560
   cctaagcctc tggtgggtta tctcctcttt gagatgtaga aaatggccag attttaataa 1620
   acgttgttac ccatgaaaaa aaaaaaaa                                    1648
<210> 45
   <211> 313
   <212> PRT
   <213> Homo sapiens
<400> 45
<210> 46
   <211> 21
   <212> ADN
   <213> Séquence artificielle
<220>
   <223> Description de la séquence artificielle:
   oligonucleotide
<400> 46
   tcgtagagca gcaggtccaa g 21